(19) Europäisches Patentamt
European Patent Office
Office européen des brevets

(11) EP 3 315 608 B1

(12) **EUROPEAN PATENT SPECIFICATION**

(45) Date of publication and mention of the grant of the patent:
**16.09.2020 Bulletin 2020/38**

(21) Application number: **16813742.0**

(22) Date of filing: **24.06.2016**

(51) Int Cl.:
***A61K 31/7088*** *(2006.01)* ***A61P 31/20*** *(2006.01)*

(86) International application number:
**PCT/CN2016/087034**

(87) International publication number:
**WO 2016/206626 (29.12.2016 Gazette 2016/52)**

(54) **SIRNA, PHARMACEUTICAL COMPOSITION AND CONJUGATE WHICH CONTAIN SIRNA, AND USES THEREOF**

SIRNA, PHARMAZEUTISCHE ZUSAMMENSETZUNG UND KONJUGAT MIT SIRNA UND VERWENDUNGEN DAVON

ARNSI, COMPOSITION PHARMACEUTIQUE ET CONJUGUÉ CONTENANT UN ARNSI, ET UTILISATIONS DE CES DERNIERS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2015 CN 201510364229**

(43) Date of publication of application:
**02.05.2018 Bulletin 2018/18**

(73) Proprietor: **Suzhou Ribo Life Science Co., Ltd.**
**Kunshan, Jiangsu 215300 (CN)**

(72) Inventors:
- **ZHANG, Hongyan**
**Kunshan**
**Jiangsu 215300 (CN)**
- **GAO, Shan**
**Kunshan**
**Jiangsu 215300 (CN)**
- **HUANG, Yuanyu**
**Kunshan**
**Jiangsu 215300 (CN)**

(74) Representative: **SSM Sandmair**
**Patentanwälte Rechtsanwalt**
**Partnerschaft mbB**
**Joseph-Wild-Straße 20**
**81829 München (DE)**

(56) References cited:
**WO-A1-2006/096018** **WO-A1-2013/061295**
**CN-A- 101 142 316** **CN-A- 101 426 912**
**CN-A- 102 140 458** **CN-A- 102 140 461**
**US-A1- 2015 174 260**

- **GAGLIONE M ET AL: "Recent progress in chemically modified siRNAs", MINI REVIEWS IN MEDICINAL CHEMISTRY, BENTHAM SCIENCE PUBL, NL, vol. 10, no. 7, 1 June 2010 (2010-06-01), pages 578-595, XP008146246, ISSN: 1389-5575, DOI: 10.2174/138955710791384036**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 315 608 B1

## Description

## FIELD OF THE INVENTION

The present invention relates to a siRNA, a pharmaceutical composition and a conjugate containing the same, and uses thereof. Specifically, the present invention relates to a siRNA for inhibiting the expression of hepatitis B virus (HBV) gene, a pharmaceutical composition and a conjugate containing said siRNA as an active ingredient, and uses of the siRNA, the pharmaceutical composition and the conjugate in preparing drugs for preventing and/or treating hepatitis B.

## BACKGROUND OF THE INVENTION

Viral hepatitis type B (also known as hepatitis type B or hepatitis B) is a class of infectious diseases, which is a serious threat to the world, especially to China. However, at present, there still exist various drawbacks in two main kinds of globally recognized drugs for the prevention/treatment of hepatitis B, interferons and nucleoside analogs. Therefore, if the generation and replication of HBV can be inhibited at gene level, thereby the virus metabolism and the infection to liver cells fundamentally reduced, it will undoubtedly be the most ideal means of the treatment of hepatitis B. For this matter, theoretically, small interfering RNA (siRNA) can inhibit or block the expression of any target gene of interest, e.g., a gene triggering a disease such as cancer, in a sequence-specific manner based on the mechanism of RNA interference (RNAi), thereby achieving the purpose of treating diseases.

Unfortunately, not many siRNA drugs have really entered into clinical application so far. One of the main reasons is that the stability of the siRNA itself is poor, easy to be degraded by the nucleases in the body (especially after administration within *in vivo* systems, the siRNA will first enter the body's blood circulation system, and the blood is rich in endogenous nucleases). To overcome this obstacle, one of the approaches is to chemically modify the siRNA to improve the stability of the siRNA in the blood. However, there still exist lack of understanding about the degradation processes and mechanisms of the siRNA in the blood in the art. It is still an empirical process that must be verified by repeated experiments to select modification sites. Thus, in order to obtain sufficient stability, a large number of (even blind, redundant) chemical modifications have to be introduced into the siRNA. As a result, although the stability of the siRNA may be increased to a certain extent by the modifications, it will definitely bring some disadvantages such as increased cost, potential cytotoxicity produced by the modified siRNA, affected biological activity and specificity of the modified siRNA and the like, due to the introduction of excessive or inappropriate modifications.

Therefore, the development of a siRNA stable in blood with a good biological activity and a considerably low cytotoxicity, which inhibits the expression of the HBV gene, has become an urgent problem to be solved.

In the granted prior application CN102140461B of the applicant, various chemical modification strategies of siRNA CA1 that specifically inhibits the HBV gene with a sense strand of 5'-CCUUGAGGCAUACUUCAAA-dTdT-3' (SEQ ID NO: 1) and an antisense strand of 5'-UUUGAAGUAUGCCUCAAGG-dTdT-3' (SEQ ID NO: 2) are studied. This study finds out that different modification strategies have completely different impacts on some parameters of the siRNA such as stability, biological activity, and cytotoxicity. For example, compared to unmodified naked siRNA CA1, siRNA CA2 carrying up to 20 chemical modifications not only exhibits poorer activity at cell level and higher cytotoxicity, but also fails to reach a stable state in the blood. In this study, five effective modification modes are confirmed. Compared to unmodified siRNA CA1, siRNA A4 yielded by one of the five modification modes shows increased stability in blood while maintaining substantially equal inhibitory activity as that of siRNA CA1. In spite of this, in consideration of various aspects including the cost and efficiency of synthesis, convenience of synthetic process, stability of siRNA, activity of siRNA and the like, it is still necessary to further simplify and optimize the modification sites of siRNA CA1, with the expectation of obtaining a more excellent siRNA. In view that previous studies in the art have indicated that different chemical modifications performed to the siRNA are likely to be especially relevant, as the saying goes, "pull one hair and the whole body is affected", i.e., even employing different modification strategies at only one single site may radically change the stability of the siRNA, it is not easy to complete the simplification and optimization as described above.

On the other hand, in addition to chemical modifications, it is also feasible to form a pharmaceutical composition with a suitable carrier or form a conjugate with a suitable conjugating molecule to further improve the stability of siRNA in the blood, to increase the targeting ability thereof, and to solve the problem of delivering siRNA *in vivo,* etc. For a siRNA intended to be used to prevent and treat hepatitis B, carriers or conjugating molecules that can confer or improve hepatic targeting ability will be very beneficial, which can largely increase the efficiency of the siRNA to inhibit the expression of HBV gene and decrease potential side effects. Furthermore, after introducing a hepatic targeting carrier or conjugating molecule, the siRNA still needs to be able to function at the target site, i.e., the encapsulation/conjugation of the carrier or the conjugating molecule cannot affect the activity of the siRNA itself (for example, it cannot affect the loading of the siRNA into the RNAi machine in cells, i.e., the RISC complex). With regard to such pharmaceutically acceptable carriers or conjugating molecules that can increase the potency of siRNA in clinical application and the siRNA pharmaceutical composition or siRNA conjugate prepared by using these carriers or conjugating molecules, there are

also urgent demands in the field.

## SUMMARY OF THE INVENTION

By conducting a detailed research on the siRNAs for inhibiting the expression of HBV gene and the modification modes thereof, the inventors found that siRNAs generated by specific modification modes exhibited excellent stability in the blood, and that a reduction in the amount of nucleotide modification decreased the costs of synthesis and production, expanded the choice of deprotecting reagents after synthesis finished, shortened the reaction time, improved the synthesis efficiency, and maintained a relatively low cytotoxicity. More importantly, the siRNA with reduced nucleotide modifications yielded by the new modification strategy of the present invention had a greater stability in the blood (the plasma) *in vitro* than that of siRNA A4 disclosed in the granted prior application CN102140461B of the applicant, and also had a greater biological activity in animals than that of siRNA A4, leading to an improved clinical application potential of the siRNA of the present invention.

Furthermore, the inventors studied a variety of pharmaceutically acceptable carriers for siRNA administration, and made an extensive and intensive research on the type, ratio (including the ratio among the internal components of the carrier and the ratio between the carrier and the siRNA of the present invention), physicochemical properties, drug loading efficiency, administration modes and doses, drug treatment time of the carrier molecules, etc. Meanwhile, the inventors also studied various types of siRNA conjugates, including the selection of pharmaceutically acceptable conjugating molecules (comprising the selection of targeting molecules as well as the linker between the targeting molecule and the siRNA of the present invention), the development and establishment of synthetic routes, study of biological activity *in vivo* and the like. Following the above systemic and thorough study, the inventors found that the siRNA of the present invention can be combined with various pharmaceutically acceptable carriers or be conjugated to various pharmaceutically acceptable conjugating molecules, achieving excellent effects *in vivo.* In particular, by forming a pharmaceutical composition of the siRNA of the present invention with a lipid mixture formed from three components of an amine- containing compound, a helper lipid and a pegylated lipid, used as a pharmaceutically acceptable carrier, the siRNA of the present invention can be specifically delivered to the liver and exhibit a greater inhibition effect on HBV mRNA while significantly reducing the administration dose. Therefore, the inventors completed the present invention.

The scope of the invention is defined in the appended claims. Any reference to "embodiment(s)", "example(s)" or "aspect(s) of the invention" in this description not falling under the scope of the claims should be interpreted as illustrative example(s) for understanding the invention.

Thus, in a first aspect, the present invention provides a siRNA that is able to specifically target the X gene region, one of the four open reading frames of HBV gene, the target mRNA sequence of said siRNA being as shown by SEQ ID NO: 3, characterized in that the sequences of the siRNA are as follows:

| | |
|---|---|
| Sense strand | 5'-CmCmUmUmGAGGCmAUmACmUmUmCmAAA-dTdT-3'; |
| Antisense strand | 5'-UfUmUfGAAGUfAUGCCUfCAAGG-dTdT-3', |

wherein the uppercase letters C, G, U, A and T represent the base composition of the nucleotide; the lowercase letter d indicates that the one nucleotide on the right side of the letter d is a deoxyribonucleotide; the lowercase letter m indicates that the 2'-hydroxyl group of the ribose group of the one nucleotide on the left side of the letter m is substituted by a methoxy group; and the lowercase letter f indicates that the 2'-hydroxyl group of the ribose group of the one nucleotide on the left side of the letter f is substituted by fluorine. For example, "Cm" consisting of the first uppercase letter C at the 5'-end of the sense strand and the letter m on the right side thereof represents a nucleotide in which the base is cytosine and the 2'-hydroxyl group of the ribose group is substituted by a methoxy group; and "Uf' consisting of the first uppercase letter U at the 5'-end of the antisense strand and the letter f on the right side thereof represents a nucleotide in which the base is uracil and the 2'-hydroxyl group of the ribose group is substituted by fluorine.

In a second aspect, the present invention provides a pharmaceutical composition comprising the siRNA according to the first aspect, as an active ingredient, and a pharmaceutically acceptable carrier.

In a third aspect, the present invention provides a siRNA conjugate obtained by conjugating the siRNA according to the first aspect to a pharmaceutically acceptable conjugating molecule.

In a fourth aspect, the present invention provides a kit comprising the siRNA according to the first aspect and/or the pharmaceutical composition according to the second aspect and/or the siRNA conjugate according to the third aspect.

In a fifth aspect, the present invention provides a use of the siRNA according to the first aspect and/or the pharmaceutical composition according to the second aspect and/or the siRNA conjugate according to the third aspect in the preparation of a drug for preventing and/or treating hepatitis B. In a sixth aspect, the present invention provides the siRNA according to the first aspect and/or the pharmaceutical composition according to the second aspect and/or the siRNA conjugate according to the third aspect for use in inhibiting the expression of HBV gene in hepatitis cells

infected with chronic HBV.

Advantageous effects

Compared to siRNA A4 in CN102140461B, the siRNA of the present invention further reduces the number of modified nucleotides, but still achieves similar or even better efficacy (similar inhibitory activity on HBV mRNA expression in cell experiments; better inhibitory activity on HBV mRNA expression and better inhibitory effect on HBsAg, the hepatitis B surface antigen, in animal experiments). More importantly, after reducing the number of modified nucleotides, the stability in the blood is improved unexpectedly (full-length siRNA fragments can maintain a higher stability in the plasma over a long period of time during *in vitro* experiments); meanwhile, due to the reduction of the amount of nucleotide modifications, the siRNA of the present invention enables a lower cost, a wider choice of technical solutions, and a higher production efficiency in the synthesis than those of siRNAA4, thereby providing a more potential siRNA in clinical application.

Further, the pharmaceutical composition and the siRNA conjugate formed by the siRNA of the present invention exhibit an excellent HBV mRNA inhibitory efficiency in hepatitis B model mice and can effectively reduce the expression of the surface antigen, showing a good therapeutic effect on chronic hepatitis B. In particular, as described above, by forming a pharmaceutical composition of the siRNA of the present invention with a lipid mixture formed from three components of an amine-containing compound, a helper lipid and a pegylated lipid, used as a pharmaceutically acceptable carrier, the siRNA of the present invention can be specifically delivered to the liver and exhibit a greater inhibition effect on HBV mRNA while significantly reducing the administration dose (in hepatitis B model mice, the pharmaceutical composition of the present invention has a higher HBV mRNA inhibitory efficiency, up to nearly 2 times or even 10 times higher than that of the pharmaceutical compositions formed by other routine carriers, even when the administration dose of the pharmaceutical composition of the present invention is only one-fifth of the pharmaceutical compositions formed by other routine carriers).

Other features and advantages of the present invention will be demonstrated in detail in the following specific embodiments.

**BRIEF DESCRIPTION OF THE DRAWINGS**

FIG. 1a-1c respectively show the following results: FIG. 1a shows the result of the Ultra Performance Liquid Chromatography (UPLC) spectrum of $(GalNAc)_3$-X2M2 duplex (after strand melting); FIG. 1b shows the Mass Spectrometry (MS) result of $(GalNAc)_3$-X2M2 sense strand; and FIG. 1c shows the MS result of $(GalNAc)_3$-X2M2 antisense strand.

FIG. 2 shows the detection results of the inhibitory activity of the test siRNAs and control siRNAs listed in Table 1 on HBV mRNA expression in HepG2.2.15 cells.

FIG. 3 shows the detection results of the stability of the test siRNAs and control siRNAs listed in Table 1 in human plasma *in vitro.*

FIG. 4 shows the detection results of the inhibitory activity of RBP131/siRNA liposome formulation encapsulated with the test siRNAs listed in Table 1 on HBV mRNA expression in C57BL/6J-Tg(Alb1HBV)44Bri/J transgenic mice.

FIG. 5 shows the detection results of the inhibitory activity of each group of test samples on HBsAg expression in the serum of M-TgHBV transgenic mice at different time points.

FIG. 6 shows the detection results of the inhibitory activity of each group of test samples on HBsAg expression in the liver of pHBV mice (HBV-transfected mice based on high-pressure injection) at day 7 and day 28.

FIG. 7 shows the detection results of the inhibitory activity of each group of test samples on HBV mRNA expression in the liver of pHBV mice (HBV-transfected mice based on high-pressure injection).

FIG. 8 shows the detection results of the inhibitory activity of each group of test samples on HBsAg expression in the serum of pHBV mice (HBV-transfected mice based on high-pressure injection).

FIG. 9 shows the detection results of the inhibitory activity of each group of test samples on HBsAg expression in

the serum of pHBV mice (HBV-transfected mice based on high-pressure injection) at different time points.

## DETAILED DESCRIPTION OF THE INVENTION

Hereinafter, specific embodiments of the present invention are described in detail. It should be understood that the specific embodiments described herein are only provided for the purpose of making illustration and explanation of the present invention, and are not intended to limit the present invention.

Hereinafter, the siRNA described in the first aspect of the present invention may also be referred to as X2M2.

I. The siRNA of the present invention

The siRNA of the present invention contains nucleotide groups as basic structural units, the nucleotide group containing a phosphate group, a ribose group and a base. The siRNA of the present invention contains modified nucleotide groups that do not cause a significant decrease or loss of the function of the siRNA to inhibit the expression of HBV gene. At present, there exist various ways to modify siRNA in the art, including backbone modification (such as phosphate group modification), ribose group modification, base modification and the like (Watts, J.K., G.F. Deleavey, and M.J. Damha, Chemically modified siRNA: tools and applications. Drug Discov Today, 2008.13(19-20): p.842-55). In some embodiments of the siRNA of the present invention, the modified nucleotide group is, but is not limited to, a nucleotide group in which the ribose group and optionally the phosphate group are modified.

In some preferred embodiments of the siRNA of the present invention, the phosphate group modification refers to phosphorothioate modification. i.e., the non-bridging oxygen atom in the phosphodiester bond is substituted by one sulfur atom so that the phosphodiester bond is replaced by a phosphorothioate diester bond. This modification can stabilize the structure of the siRNA while maintaining a high specificity and a high affinity of base pairing.

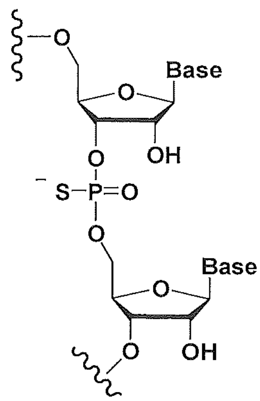

Phosphorothioate modification

In some preferred embodiments of the siRNA of the present invention, the ribose group modification refers to that the 2'-hydroxyl group in the ribose group is substituted by a methoxy group or fluorine. Upon introducing certain substituents such as a methoxy group or fluorine at 2'-hydroxyl position of the ribose group, ribonucleases in the blood cannot easily digest nucleic acid, thereby improving the stability of the nucleic acid and allowing the nucleic acid to have stronger resistance against nuclease hydrolysis.

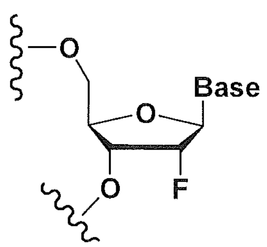 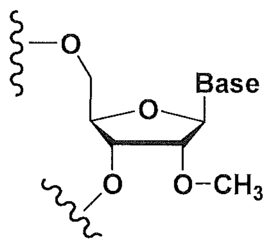

2'-fluoro modification 2'-methoxy modification

In the siRNA of the present invention, the 3'-end of both the sense strand and the antisense strand is linked with an overhang dTdT. According to a preferred embodiment of the present invention, the phosphodiester bond between the overhang dTdT at the 3'-end of the sense strand and/or the antisense strand is modified by phosphorothioate modification. Hereinafter, the siRNA in which the phosphodiester bonds between the overhang dTdT at the 3'-end of both the sense strand and the antisense strand are modified by phosphorothioate modification may be called X2M2(S++); the siRNA in which no phosphodiester bonds between the overhang dTdT at the 3'-end of the sense strand and the antisense strand are modified by phosphorothioate modification may be called X2M2(S--); the siRNA in which only the phosphodiester bond between the overhang dTdT at the 3'-end of the sense strand is modified by phosphorothioate modification may be called X2M2(S+-); and the siRNA in which only the phosphodiester bond between the overhang dTdT at the 3'-end of the antisense strand is modified by phosphorothioate modification may be called X2M2(S-+). In addition, unless the context specifically indicates otherwise, when referring to siRNA X2M2, all of X2M2(S++), X2M2(S--), X2M2(S+-) and X2M2(S-+) listed here are intended to be covered.

The skilled in the art appreciate that the siRNA of the present invention, for example, X2M2(S++), X2M2(S--), X2M2(S+-) and X2M2(S-+), can be obtained by common methods for preparing siRNA in the art, e.g., solid phase synthesis and liquid phase synthesis. Therein, commercial customization services have already been available for solid phase synthesis, and Suzhou Ribo Life Science Co., Ltd. also has the ability to provide such solid phase synthesis services. Modified nucleotide groups can be introduced into the siRNA of the present invention by using a nucleotide monomer having a corresponding modification, wherein the methods for preparing a nucleotide monomer having a corresponding modification and the methods for introducing a modified nucleotide group into a siRNA are also well-known for the skilled in the art.

II. The pharmaceutical composition of the present invention

The pharmaceutical composition of the present invention contains the siRNA of the present invention and a pharmaceutically acceptable carrier. The pharmaceutically acceptable carrier may be a carrier conventionally used in the field of siRNA administration, for example, but is not limited to, magnetic nanoparticles such as $Fe_3O_4$ and $Fe_2O_3$, carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/ glycolic acid) copolymer (PLGA), poly(2-aminoethyl ethylene phosphate) (PPEEA) and poly(2-dimethylaminoethyl methacrylate) (PDMAEMA), and derivatives thereof. In the pharmaceutical composition of the present invention, there are no special requirements of the contents of the siRNA and the pharmaceutically acceptable carrier. Generally, the weight ratio of the siRNA to the pharmaceutically acceptable carrier is 1: (1-50).

The pharmaceutical composition of the present invention may also contain other pharmaceutically acceptable excipients, which may be one or more of the various formulations or compounds conventionally employed in the art. For example, said other pharmaceutically acceptable excipients may include at least one of a pH buffer, a protective agent and an osmotic pressure regulator. The pH buffer may be tris(hydroxymethyl) aminomethane hydrochloride buffer solution with a pH of 7.5-8.5, and/or phosphate buffer solution with a pH of 5.5-8.5, preferably phosphate buffer solution with a pH of 5.5-8.5. The protective agent may be at least one of inositol, sorbitol, sucrose, trehalose, mannose, maltose, lactose and glucose. The content of the protective agent may be from 0.01wt% to 30wt% on the basis of the total weight of the pharmaceutical composition. The osmotic pressure regulator may be sodium chloride and/or potassium chloride. The content of the osmotic pressure regulator allows the osmotic pressure of the pharmaceutical composition to be 200-700 milliosmol/kg. Depending on the desired osmotic pressure, the skilled in the art can readily determine the content of the osmotic pressure regulator.

According to some embodiments of the present invention, the pharmaceutical composition may be a liquid formulation, for example, an injection solution; or a lyophilized powder for injection, which is mixed with a liquid excipient to form a liquid formulation when performing administration. The liquid formulation may, but is not limited to, be used for subcutaneous, intramuscular or intravenous administration by injection, and also may, but is not limited to, be administrated to the lung by spray, or to other organ tissues (such as the liver) through the lung by spray. Preferably, the pharmaceutical composition is used for intravenous administration by injection.

In a preferred embodiment of the pharmaceutical composition of the present invention, the pharmaceutical composition may be in the form of a liposome formulation. In a more preferred embodiment, the pharmaceutically acceptable carrier used in the liposome formulation comprises an amine-containing transfection compound (hereinafter also referred to as an amine-containing compound), a helper lipid and/or a pegylated lipid. Therein, the amine-containing compound, the helper lipid and the pegylated lipid may be respectively selected from one or more of the amine-containing transfection compounds or the pharmaceutically acceptable salts or derivatives thereof, the helper lipids and the pegylated lipids described in CN201180060664.1.

In particular, the amine-containing compound may be a compound represented by Formula I or a pharmaceutically acceptable salt thereof described in CN201180060664.1:

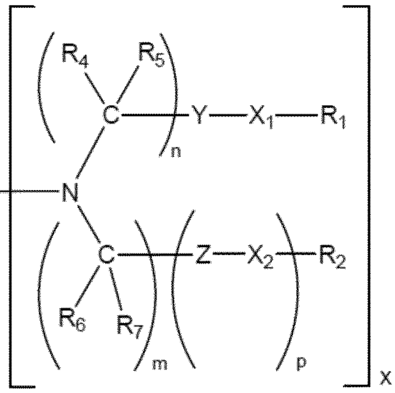
Formula I

wherein:

each of $X_1$ and $X_2$ is independently selected from O, S, N-A or C-A, wherein A is hydrogen or a $C_1$-$C_{20}$ hydrocarbon chain;
each of Y and Z is independently selected from C=O, C=S, S=O, CH-OH or $SO_2$;
each of $R_1$, $R_2$, $R_3$, $R_4$, $R_5$, $R_6$ and $R_7$ is independently selected from hydrogen; a cyclic or an acyclic, substituted or unsubstituted, branched or unbranched aliphatic group; a cyclic or an acyclic, substituted or unsubstituted, branched or unbranched heteroaliphatic group; a substituted or unsubstituted, branched or unbranched acyl group; a substituted or unsubstituted, branched or unbranched aryl group, a substituted or unsubstituted, branched or unbranched heteroaryl group;
x is an integer having the value between 1 and 10;
n is an integer having the value between 1 and 3, m is an integer having the value between 0 and 20,
p is an integer having the value of 0 or 1, wherein if m=p=0, then $R_2$ is hydrogen,

with the further proviso that if at least one of n or m has the value of 2, then $R_3$ and nitrogen in Formula I form a structure as represented by Formula II or Formula III:

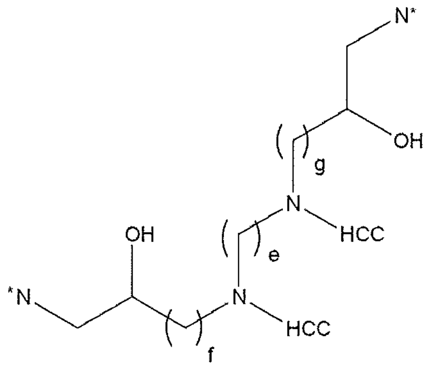
Formula II;

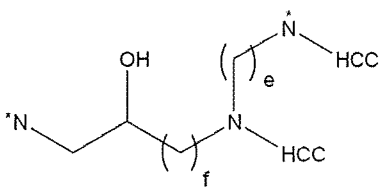
Formula III,

wherein each of g, e and f is independently an integer having the value between 1 and 6, "HCC" symbolizes a hydrocarbon chain, and each * indicates the nitrogen atom in Formula I.

Preferably, the amine-containing compound may be amine-containing compound 72 or amine-containing compound 87 as described in CN201180060664.1, as follows:

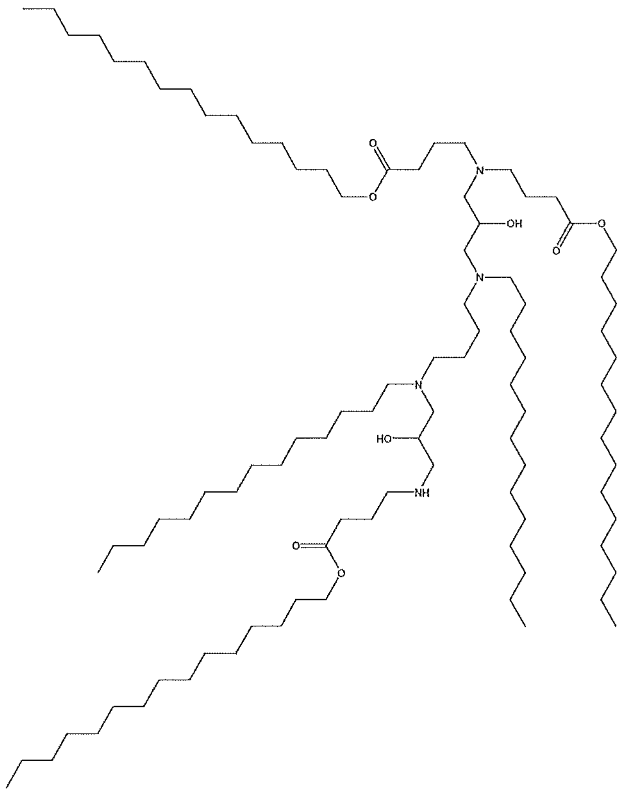

72 ;

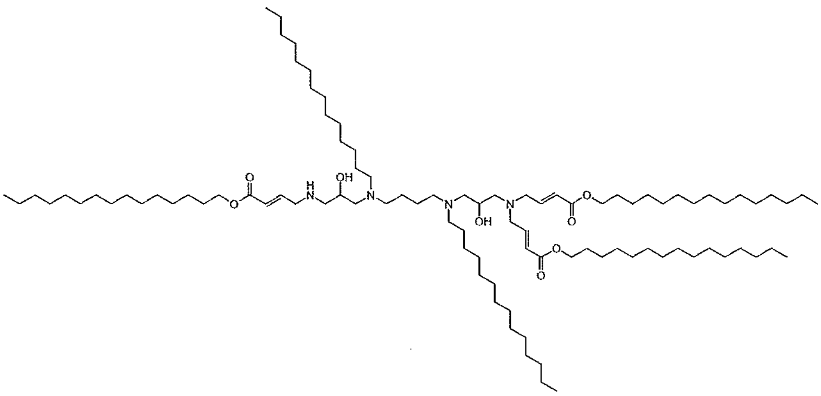

87 .

Preferably, the helper lipid may be cholesterol or analogs and derivatives thereof, and the like.

Preferably, the pegylated lipid may be 1,2-dipalmitoyl-*sn*-glycero-3-phosphoethanolamine-N-[methoxy(polyethylene glycol)-2000].

In a more preferred embodiment of the pharmaceutical composition of the present invention, the pharmaceutically acceptable carrier used comprises all of the three components of the amine-containing compound, the helper lipid and the pegylated lipid as described above, the three components forming a lipid mixture. In this preferred embodiment, the molar percentages of the amine-containing compound, the helper lipid, and the pegylated lipid in the pharmaceutically acceptable carrier may be 19.7%-80% for the amine-containing compound, 19.7%-80% for the helper lipid, and 0.3%-50% for the pegylated lipid. More preferably, the molar percentages of the amine-containing compound, the helper lipid, and the pegylated lipid in the pharmaceutically acceptable carrier may be 50%-70% for the amine-containing compound, 20%-40% for the helper lipid, and 3%-20% for the pegylated lipid.

Methods for preparing pharmaceutical compositions in the form of functional liposome formulations by using the above amine-containing compounds, helper lipids, pegylated lipids and siRNAs can also refer to CN201180060664.1. For such preparation methods, the molar percentages of the amine-containing compound, the helper lipid, and the

pegylated lipid in the pharmaceutically acceptable carrier in the pharmaceutical composition may be set as 19.7%-80% for the amine-containing compound, 19.7%-80% for the helper lipid, and 0.3%-50% for the pegylated lipid. More preferably, the molar percentages of the amine-containing compound, the helper lipid, and the pegylated lipid in the pharmaceutically acceptable carrier in the pharmaceutical composition may be set as 50%-70% for the amine-containing compound, 20%-40% for the helper lipid, and 3%-20% for the pegylated lipid.

The liposome particles formed by the siRNA of the present invention and the above lipid mixture have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In the pharmaceutical composition in the form of liposome formulation, the weight ratio (weight/weight ratio) of the siRNA of the present invention to total lipids, e.g., the amine-containing compounds, the helper lipids and/or the pegylated lipids, ranges from about 1: 1 to about 1: 50, from about 1: 1 to about 1: 30, from about 1: 3 to about 1: 20, from about 1: 4 to about 1: 18, from about 1: 5 to about 1: 17, from about 1: 5 to about 1: 15, from about 1: 5 to about 1: 12, from about 1: 6 to about 1: 12, or from about 1: 6 to about 1: 10, for example, the weight ratio of the siRNA of the present invention to total lipids is about 1: 5, 1: 6, 1: 7, 1: 8, 1: 9, 1: 10, 1: 11, 1: 12, 1: 13, 1: 14, 1: 15, 1: 16, 1: 17 or 1: 18.

III. The siRNA conjugate of the present invention

The siRNA conjugate of the present invention is obtained by conjugating the siRNA of the present invention to a pharmaceutically acceptable conjugating molecule, which comprises a pharmaceutically acceptable targeting molecule and an optional linker. In the embodiments of the present invention, the siRNA may be non-covalently conjugated to the conjugating molecule, but preferably, the siRNA is covalently conjugated to the conjugating molecule. To reduce the effect of conjugation on siRNA activity, the conjugating site between the siRNA and the conjugating molecule can be at the 3'-end or 5'-end of the siRNA sense strand, or at the 5'-end of the antisense strand. In some embodiments, the conjugating site between the siRNA and the conjugating molecule may also within the internal sequence of the siRNA, in addition to the 3'-end or 5'-end.

The siRNA and the conjugating molecule can be linked by acid-labile or reducible chemical bonds, and these chemical bonds can be degraded under the acidic environment of cell endosomes, thereby making the siRNA free. For non-degradable conjugating means, the conjugating molecule can be linked to the sense strand of the siRNA, thereby minimizing the effect of conjugation on siRNA activity.

The pharmaceutically acceptable targeting molecule may be a targeting molecule conventionally used in the field of siRNA administration, such as, but is not limited to, one or more of the following targeting molecules or derivatives thereof: lipophilic molecules, such as cholesterol, bile acids, vitamins such as vitamin E, lipid molecules of different chain lengths; polymers, such as polyethylene glycol; polypeptides, such as cell-penetrating peptide; aptamers; antibodies; quantum dots; saccharides, such as lactose, polylactose, mannose, galactose, N-acetylgalactosamine (GalNAc); folate; or receptor ligands expressed in hepatic parenchymal cells, such as asialoglycoprotein, asialo-sugar residue, lipoproteins (such as high density lipoprotein, low density lipoprotein and the like), glucagon, neurotransmitters (such as adrenaline), growth factors, transferrin and the like.

In some preferred embodiments of the siRNA conjugate of the present invention, the pharmaceutically acceptable targeting molecule may be selected from one or more of the following molecules: asialoglycoprotein, such as asialoorosomucoid (ASOR); ligands for asialoglycoprotein receptor (ASGPR), such as lactose, polylactose, mannose, galactose and N-acetylgalactosamine.

In some more preferred embodiments of the siRNA conjugate of the present invention, the pharmaceutically acceptable targeting molecule may be galactose or N-acetylgalactosamine, wherein the galactose or N-acetylgalactosamine molecules can be mono-, bi-, tri-, or tetra-valent. It should be understood that the terms mono-, bi-, tri-, or tetravalent described herein respectively mean that the molar ratio of the siRNA molecule to the galactose or N-acetylgalactosamine molecule in the siRNA conjugate is 1: 1, 1: 2, 1: 3 or 1: 4 after the siRNA molecule forms a siRNA conjugate with the conjugating molecule containing galactose or N-acetylgalactosamine molecules as targeting molecules. More preferably, the pharmaceutically acceptable targeting molecule is N-acetylgalactosamine. Still more preferably, when the siRNA of the present invention is conjugated to a conjugating molecule comprising N-acetylgalactosamine, the N-acetylgalactosamine molecule is trivalent or tetravalent. Even more preferably, when the siRNA of the present invention is conjugated to a conjugating molecule comprising N-acetylgalactosamine, the N-acetylgalactosamine molecule is trivalent.

When the siRNA of the present invention is conjugated to a conjugating molecule, the conjugating molecule can be linked to the siRNA molecule via an appropriate linker, and the appropriate linker can be selected by the skilled in the art according to the specific type of the targeting molecule. In some embodiments, when the targeting molecule is N-acetylgalactosamine, a suitable linker may be of the following structure: $-(L^A)_nL^C-L^B-$, wherein,

n is an integer having the value between 1 and 3;
$L^A$ is a chain moiety comprising amide bond, which is linked to the N-acetylgalactosamine molecule and the $L^C$ moiety through ether bond, and the structure thereof is as follows:

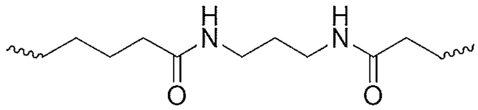

;

$L^B$ is a chain moiety comprising amide bond, which is linked to the $L^C$ moiety through amide bond and linked to the siRNA moiety through phosphoester bond, and the structure thereof is as follows:

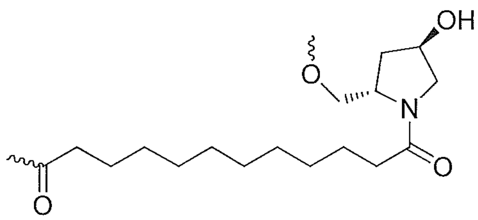

;

and
$L^C$ is a bivalent to tetravalent linking group based on hydroxymethyl aminomethane, dihydroxymethyl aminomethane or trihydroxymethyl aminomethane, one end of which may be linked to 1-3 $L^A$ moieties through ether bond via oxygen atom, and thus be lined to 1-3 N-acetylgalactosamine molecule(s) via the $L^A$ moiety; and the other end of which is linked to the $L^B$ moiety through amide bond via nitrogen atom.

In a preferred embodiment, when n=3 and $L^C$ is a tetravalent linking group based on trihydroxymethyl aminomethane, the structure of the siRNA conjugate formed by linking N-acetylgalactosamine molecules with a siRNA molecule via -$(L^A)_3$-trihydroxymethyl aminomethane-$L^B$- as a linker is as follows:

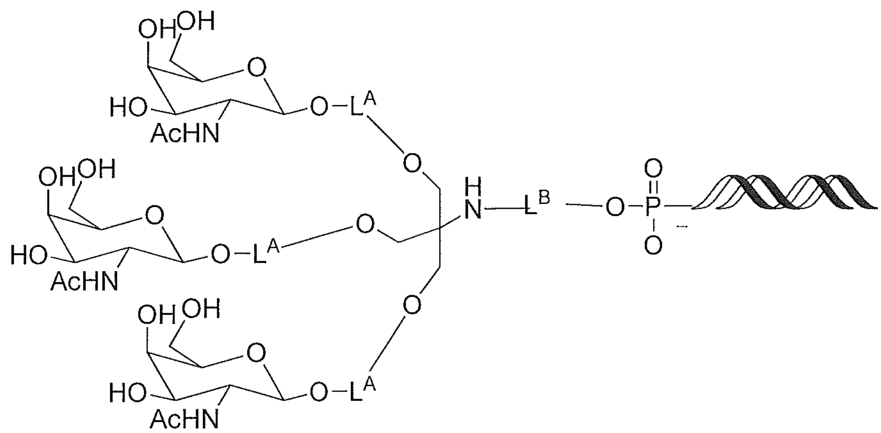

.

Likewise, the conjugating site between the siRNA and the conjugating molecule can be at the 3'-end or 5'-end of the siRNA sense strand, or at the 5'-end of the antisense strand, or within the internal sequence of the siRNA.

Thus, most preferably, the 3'-end of the sense strand of the siRNA of the present invention is covalently conjugated to three N-acetylgalactosamine (GalNAc) molecules via a linker - $(L^A)_3$-trihydroxymethyl aminomethane-$L^B$- to obtain a siRNA conjugate in which the molar ratio of the siRNA molecule to the GalNAc molecule is 1: 3 (hereinafter this siRNA conjugate may be referred to as $(GalNAc)_3$-X2M2), and the structure of this siRNA conjugate is as follows:

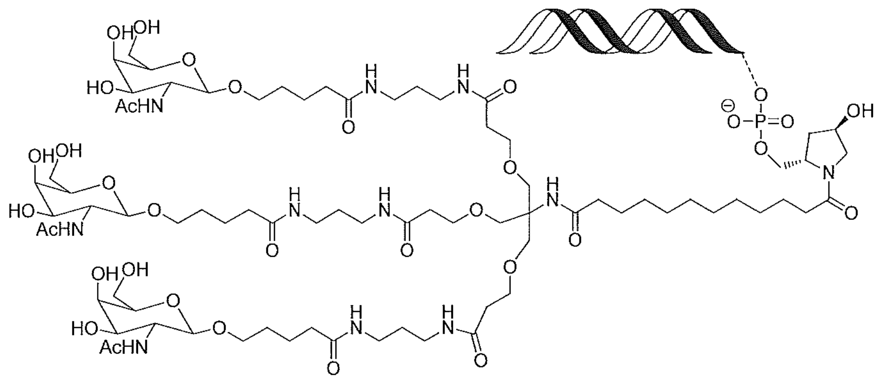

.

For the siRNA conjugate of the present invention, it may also be used in combination with other pharmaceutically acceptable excipients, which may be one or more of various formulations or compounds conventionally employed in the art. Please see the above description of the pharmaceutical composition of the present invention for details.

IV. The kit of the present invention

In some embodiments of the kit of the present invention, one container may be used to provide the siRNA, and at least another container used to provide the pharmaceutically acceptable carrier and/or excipient. In addition to the siRNA and the pharmaceutically acceptable carrier and/or excipient, the kit may also contain other ingredients such as a stabilizer or a preservative. Said other ingredients may be included in the kit but provided in a container different from the containers for providing the siRNA and the pharmaceutically acceptable carrier and/or excipient. In these embodiments, the kit may comprise an instruction for mixing the siRNA with the pharmaceutically acceptable carrier and/or excipient or other ingredients.

In some other embodiments of the kit of the present invention, the siRNA conjugate may be stored in one container; and there may be no other container or at least another container, for providing or not providing the pharmaceutically acceptable excipient. In addition to the siRNA conjugate and optional pharmaceutically acceptable excipient, the kit may also contain other ingredients such as a stabilizer or a preservative. Said other ingredients may be included in the kit but provided in a container different from the containers for providing the siRNA conjugate and optional pharmaceutically acceptable excipient. In these embodiments, the kit may comprise an instruction for mixing the siRNA conjugate with the pharmaceutically acceptable excipient (under the circumstance that there is excipient) or other ingredients.

In the kit of the present invention, the siRNA and the pharmaceutically acceptable carrier and/or excipient as well as the siRNA conjugate and optional pharmaceutically acceptable excipient may be provided in any form, such as in a liquid form, dried form or lyophilized form. Preferably, the siRNA and the pharmaceutically acceptable carrier and/or excipient as well as the siRNA conjugate and optional pharmaceutically acceptable excipient are substantially pure and/or sterilized. Sterilized water may be provided optionally in the kit of the present invention.

V. The use of the siRNA, the pharmaceutical composition and the siRNA conjugate of the present invention

By administrating the siRNA and/or the pharmaceutical composition and/or the siRNA conjugate of the present invention to a patient in need thereof, the purpose of treating hepatitis B can be achieved by the mechanism of RNA interference. Thus, the siRNA and/or the pharmaceutical composition and/or the siRNA conjugate of the present invention can be used to prevent and/or treat hepatitis B, or can be used for the preparation of a drug for preventing and/or treating hepatitis B.

As used herein, the term "administration/administrate" refers to placing the siRNA or the pharmaceutical composition or the siRNA conjugate into a subject by a method or route where at least, in part, the siRNA or the pharmaceutical composition or the siRNA conjugate is located at a desired site to achieve a desired effect. The administration routes suitable for the method of the present invention include topical administration and systemic administration. In general, topical administration results in that more siRNA or pharmaceutical composition or siRNA conjugate is delivered to a particular site as compared to the entire body of the subject; while systemic administration results in that the siRNA or pharmaceutical composition or siRNA conjugate is delivered to basically the whole body of the subject. Considering that the present invention is intended to provide means for the prevention and/or treatment of hepatitis B, an administration mode that can deliver drugs to the liver is preferable.

Any suitable route known in the art can be used to administrate the subject, including, but is not limited to, oral

or parenteral routes, including intravenous administration, intramuscular administration, subcutaneous administration, transdermal administration, endotracheal administration (aerosol), pulmonary administration, nasal administration, rectal administration and topical administration (including buccal administration and sublingual administration).

The application dosage of the siRNA or the pharmaceutical composition or the siRNA conjugate of the present invention can be a conventional dose in the art, which may be determined according to various parameters, especially the age, weight and gender of the subject. Toxicity and therapeutic efficacy can be determined by standard pharmaceutical procedures in cell cultures or experimental animals, e.g., the measurement of $LD_{50}$ (the dose that is lethal to 50% of the population) and $ED_{50}$ (the dose that can cause 50% of the maximum response intensity in the quantitative response, or the dose that can cause a positive response in 50% of the experimental subjects in the qualitative response). The dose ratio between toxic and therapeutic effects is the therapeutic index, which can be expressed as the ratio, $LD_{50}/ED_{50}$. The siRNA or pharmaceutical composition or siRNA conjugate which exhibits a large therapeutic index is preferred. The data obtained from cell culture assays and animal studies can be used in formulating a range of dosage for human use.

When administrating the pharmaceutical composition or the siRNA conjugate of the present invention, for example, for C57BL/6J or C3H/HeNCrlVr mice, either male or female, with an age of 6 to 12 weeks and a weight of 18 to 25 g, when calculated by the amount of the siRNA contained in the pharmaceutical composition or the siRNA conjugate: (i) for a pharmaceutical composition formed by a siRNA and a pharmaceutically acceptable carrier, the dosage of siRNA thereof may be from 0.001 to 50 mg/kg body weight, preferably from 0.01 to 10 mg/kg body weight, more preferably from 0.05 to 5 mg/kg body weight, and most preferably from 0.1 to 3 mg/kg body weight; (ii) for a siRNA conjugate formed by a siRNA and a pharmaceutically acceptable conjugating molecule, the dosage of siRNA thereof may be from 0.001 to 100 mg/kg body weight, preferably from 0.01 to 50 mg/kg body weight, more preferably from 0.05 to 20 mg/kg body weight, and most preferably from 0.1 to 10 mg/kg body weight. The above dosages can be referred to when administrating the siRNA of the present invention.

By introducing the siRNA and/or the pharmaceutical composition and/or the siRNA conjugate of the present invention into hepatitis cells infected with chronic HBV, the purpose of inhibiting the expression of HBV gene in the hepatitis cells infected with chronic HBV may also be achieved by the mechanism of RNA interference. In a preferred embodiment, the cells are HepG2.2.15 cells.

## EXAMPLES

Hereinafter, the present invention will be described in detail by way of examples. Unless specifically illustrated, all of the reagents and media used in the following examples are commercially available, and all of the operations such as nucleic acid electrophoresis, real-time PCR and the like are carried out according to conventional protocols. For example, the operations can be performed as described in Molecular Cloning (Cold Spring Harbor Laboratory Press (1989)).

HepG2.2.15 cells were purchased from ATCC, cultured with DMEM complete medium (Gibco) containing 10% fetal bovine serum (FBS, Gibco), 2 mM L-glutamine (Gibco) and 380 μg/ml G418 in an incubator containing 5% $CO_2$/95% air at 37 °C.

When using the siRNAs against the X gene region of HBV or the non-specific siRNAs as negative controls synthesized in Preparation Example 1 (all of which were synthesized by Suzhou Ribo Life Science Co., Ltd.) to transfect cells, Lipofectamine™ 2000 (Invitrogen) was used as a transfection reagent. The specific procedures thereof may refer to the instruction provided by the manufacturer.

All the data were expressed as "mean ± standard deviation". The homogeneity test of variances was performed using Levene's test at first. The data of homoscedasticity were tested by using one-way ANOVA, and then Dunnett's test if the results show the presence of difference; while the data of heteroscedasticity were tested by using Dunnett's test after a rank transformation. The statistical software was SPSS (version 13.0). $P < 0.05$ indicated a significant difference between the groups.

### Preparation Example 1: Screening and Synthesis of siRNA

The siRNA sequences listed in Table 1 below were screened out through combined evaluation and analysis of various modification modes and modification sites. Therein, NC-pos, NC-bas and NC were negative controls that had no inhibitory effect on HBV gene, hereinafter also referred to as control siRNAs; the rest siRNAs were siRNAs that specifically targeted the X gene region of HBV, hereinafter also referred to as test siRNAs.

The siRNAs listed in Table 1 were obtained by conventional solid phase synthesis method by Suzhou Ribo Life Science Co., Ltd. Mixtures of equimolar amounts of the sense strand and the antisense strand were dissolved with an annealing salt solution followed by conventional annealing to form siRNA duplexes.

Table 1 Sequences of siRNA

| siRNA | Sense strand (5'→3') | Antisense strand (5'→3') |
|---|---|---|
| NC | UUCUCCGAACGUGUCACGUdTdT (SEQ ID NO: 4) | ACGUGACACGUUCGGAGAAdTdT (SEQ ID NO: 5) |
| NC-pos | UmUmCmUmCCGAAmCGmUGmUmCmAmCGUdTdT | AfCmGfUGACAfCGfUUCGfGAGAAdTdT |
| NC-bas | UmUmCmUmCmCmGAACmGUmGUmCmACmGUmdTdT | ACmGUfGACfACfGUfUfCfGGAGAAdTdT |
| CA1 | CCUUGAGGCAUACUUGCAAAdTdT | UUUGAAGUAUGCCUCAAGGdTdT |
| CA2 | CmCUmUGmAGmGCmAUmACmUUmCAmAAmdTdT | UmUUmGAmAGmUAmUGmCCmUCmAAmGGmdTdT |
| A4 | CmCmUmUmGAGGCmAUmACmUmUmCmAAAdTdT | UfUmUfGAAGUfAUfGCCUfCAAGGdTdT |
| 1d | CmCmUmUmGAGGCmAUmACmUmUmCmAAAdTdT | UUmUfGAAGUfAUfGCCUfCAAGGdTdT |
| 2d | CmCmUmUmGAGGCmAUmACmUmUmCmAAAdTdT | UfUUfGAAGUfAUfGCCUfCAAGGdTdT |
| 3d | CmCmUmUmGAGGCmAUmACmUmUmCmAAAdTdT | UfUmUGAAGUfAUfGCCUfCAAGGdTdT |
| 8d | CmCmUmUmGAGGCmAUmACmUmUmCmAAAdTdT | UfUmUfGAAGUAUfGCCUfCAAGGdTdT |
| 14d | CmCmUmUmGAGGCmAUmACmUmUmCmAAAdTdT | UfUmUfGAAGUfAUfGCCUCAAGGdTdT |
| X2M2 (S++) | CmCmUmUmGAGGCmAUmACmUmUmCmAAAdT-s-dT | UfUmUfGAAGUfAUGCCUfCAAGGdT-s-dT |
| X2M2 (S--) | CmCmUmUmGAGGCmAUmACmUmUmCmAAAdTdT | UfUmUfGAAGUfAUGCCUfCAAGGdTdT |

Note: the uppercase letters C, G, U, A and T represent the base composition of the nucleotide; the lowercase letter d indicates that the one nucleotide on the right side of the letter d is a deoxyribonucleotide; the lowercase letter m indicates that the 2'-hydroxyl group of the ribose group of the one nucleotide on the left side of the letter m is substituted by a methoxy group; the lowercase letter f indicates that the 2'-hydroxyl group of the ribose group of the one nucleotide on the left side of the letter f is substituted by fluorine; and the lowercase letter s indicates that the phosphodiester bond between the overhang dTdT at the 3'-end is replaced by a phosphorothioate diester bond.

**Preparation Example 2: Synthesis of RBP131/siRNA liposome formulation**

Three dry powder lipid compounds, i.e., the amine-containing compound, the helper lipid and the pegylated lipid, were suspended in ethanol at a molar ratio of 59: 29: 12 and mixed. The total mass concentration of the three lipid compounds was about 8.85 mg/ml. The synthesized siRNAs listed in Table 1 were dissolved in 200 mM sodium acetate (pH 5.2) solution so that the concentration of the siRNAs was 0.2 mg/ml. The resulting lipid ethanol solution and siRNA

aqueous sodium acetate solution were mixed very rapidly at a volume ratio of 1: 3. The specific composition of the liposome formulation obtained after mixing were described in Table 2.

Table 2 Composition of liposome formulation

| Composition | Content |
|---|---|
| Amine-containing compound (compound 87 in CN201180060664.1) | 1.52 mg/ml |
| Cholesterol | 0.18 mg/ml |
| Pegylated lipid (1,2-dipalmitoyl-*sn*-glycero-3-phosphoethanolamine-N-[methoxy(pol yethylene glycol)-2000]) | 0.51 mg/ml |
| siRNA | 0.15 mg/ml |
| Buffer salts | 50 mM sodium acetate |
| Solution | 25% ethanol |

The liposome formulation obtained after mixing, i.e., the composition of the amine- containing compound, cholesterol, pegylated lipid and the siRNA, was incubated at about 50 °C for 10 minutes. After incubation, ultrafiltration was performed with 100KDa hollow fiber column and PBS at pH 7.4 as ultrafiltration exchange solution using the KrosFlo® tangential flow system. The formulation can be concentrated or diluted to obtain a desired siRNA concentration during ultrafiltration. The formulation after ultrafiltration was sterilized by filtering with a 0.22 μm filter. The lipid mixture, consisting of amine-containing compound 87, cholesterol, and 1,2-dipalmitoyl-*sn*-glycero-3-phosphoethanolamine-N-[methoxy(polyethylene glycol)-2000], was referred to as RBP131. The resulting RBP131/siRNA liposome formulation was stored at 4 °C prior to use. The relevant physiochemical properties thereof were detected, and the results thereof were shown in Table 3.

Table 3 Physiochemical parameters of RBP131/siRNA liposome formulation

| Indication detected | Result |
|---|---|
| pH | 7.4 |
| Encapsulation efficiency (%) | 95.9 |
| siRNA concentration (mg/ml) | 0.14 |
| Particle size (nm) | 69.25 |
| PDI | 0.121 |
| Osmotic pressure (mOsm/kg) | 300.5 |

In addition, liposome formulation RBP130/siRNA was prepared in the same manner as described above, with the exception that the amine-containing compound was replaced with compound 72 in CN201180060664.1. The physiochemical parameters of this liposome formulation RBP130/siRNA were similar to those shown in Table 3.

**Preparation Example 3: Synthesis of (GalNAc)$_3$-X2M2 conjugate**

(1) Synthesis of (GalNAc)$_3$ conjugating molecule

Compound 30, i.e., the conjugating molecule containing the abovementioned linker - $(L^A)_3$-trihydroxymethyl aminomethane-$L^B$- and N-acetylgalactosamine molecule as targeting molecule (wherein each $L^A$ can be linked to one N-acetylgalactosamine molecule so that one linker can be linked to three N-acetylgalactosamine molecules), was synthesized according to the preparation method described in WO2014025805A1. This conjugating molecule can also be referred to herein as (GalNAc)$_3$ conjugating molecule, and the structure of compound 30 was as follows:

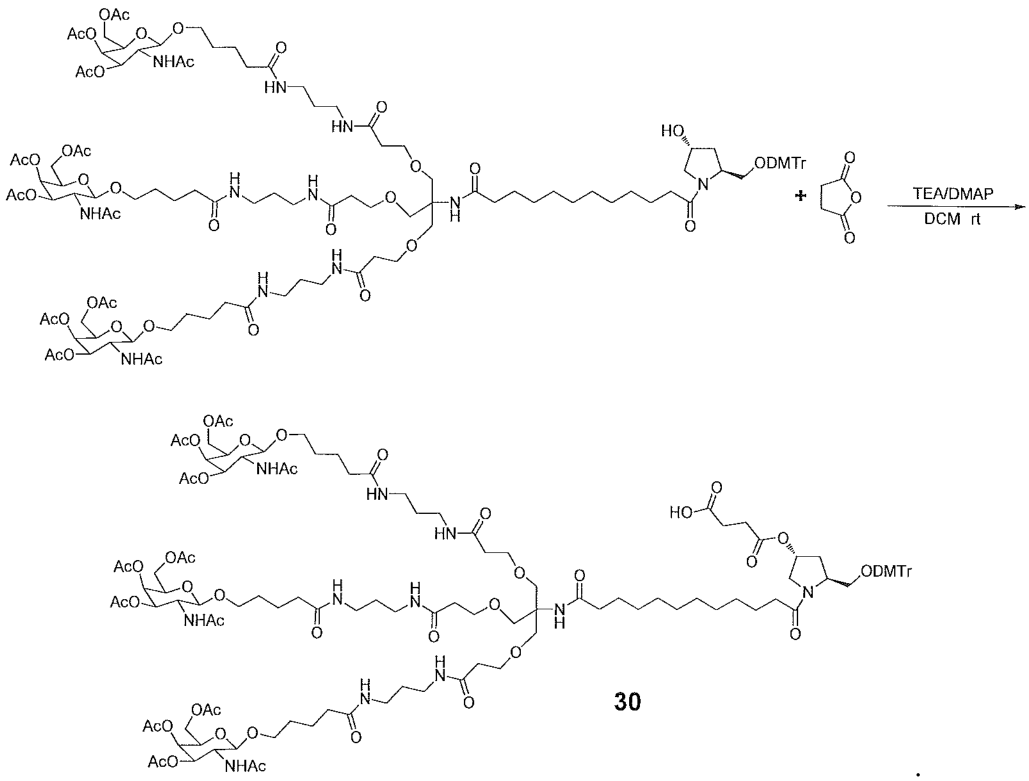


30

(2) Linking the $(GalNAc)_3$ conjugating molecule to a solid phase support

$(GalNAc)_3$ conjugating molecule (50 mg, 0.020 mmol) and HBTU (9.1 mg, 0.024 mmol) were weighed and added to a reaction flask, then 2ml DMF and DIEA (10.8 μl, 0.06 mmol) were added, and the reaction flask was gently shaken at room temperature for 2 to 3 min to make all of them fully dissolved, and macroporous aminomethyl resin (50 mg, Nankai HECHENG S&T Co., Ltd, Cat. No. HC4025-1/HC4025-2) was added thereto. The reaction flask was placed on a shaker and shaken overnight at 30 °C at 250 rpm/min. After the reaction finished, filtration was performed and the resulting solid was washed once with 2 ml methanol/dichloromethane (at a volume ratio of 1: 9) and washed again with 2 ml ether, and then the solid was dried by suction. As a result, a solid crude product of about 66 mg was obtained.

The resulting solid crude product was added into 1.5 ml acetic anhydride/pyridine (at a volume ratio of 1: 3) and placed on a shaker to be shaken at room temperature for 30 min. Filtration was performed and the resulting solid was washed once with 2 ml methanol/dichloromethane (at a volume ratio of 1: 9) and washed again with 2 ml ether, and then the solid was dried by suction. A solid final product, i.e., a solid phase support linked to $(GalNAc)_3$ conjugating molecule, of about 56 mg was yielded.

(3) Synthesis of $(GalNAc)_3$-X2M2 conjugate

The solid phase support linked to $(GalNAc)_3$ conjugating molecule prepared according to the above procedures was used to synthesize the sense strand S of $(GalNAc)_3$-X2M2 by the method of phosphoramidite nucleic acid solid phase synthesis; and a dT solid phase support (GE Healthcare, Cat. No. 17-5253-83) was used to synthesize the antisense strand AS of $(GalNAc)_3$-X2M2.

The sequences were shown in Table 4 below.

Table 4 Sequence composition of $(GalNAc)_3$-X2M2

| Sequence name | | Sequence composition (5'-3') |
|---|---|---|
| $(GalNAc)_3$-X2M2 | S | 5'-CmCmUmUmGAGGCmAUmACmUmUmCmAAAdTdT-$(GalNAc)_3$-3' |
| | AS | 5'-UfUmUfGAAGUfAUGCCUfCAAGGdTdT-3' |

(continued)

| Sequence name | Sequence composition (5'-3') |
|---|---|
| Note: the lowercase letter d indicates that the one nucleotide on the right side of the letter d is a deoxyribonucleotide; the lowercase letter m indicates that the 2'-hydroxyl group of the ribose group of the one nucleotide on the left side of the letter m is substituted by a methoxy group; the lowercase letter f indicates that the 2'-hydroxyl group of the ribose group of the one nucleotide on the left side of the letter f is substituted by fluorine; and $(GalNAc)_3$ represents $(GalNAc)_3$ conjugating molecule, which is compound 30. | |

The synthesis of $(GalNAc)_3$-X2M2 was carried out on a 1 μmol scale, and the specific synthesis steps were as follows.

Synthesis: a nucleic acid solid phase synthesizer (American ABI Co., model: ABI394 synthesizer) was used to complete the elongation of the nucleic acid sequence from the 3'-end to the 5'-end via automatic cycle, wherein each cycle included four steps of reactions (deprotection, coupling, capping, and oxidation). All the conventional nucleoside monomers, methoxy monomers, and fluorinated monomers used for the synthesis of $(GalNAc)_3$-X2M2 were formulated as 0.1 M acetonitrile solutions, and standard deprotection, coupling, capping, oxidation reagents in solid phase synthesis were used. The coupling time of the monomers was 12 min. $(GalNAc)_3$ conjugating molecule was linked to the siRNA via phosphodiester bond, and the molar ratio of the siRNA molecule to the N-acetylgalactosamine molecule in the finally resulting $(GalNAc)_3$-X2M2 conjugate was 1: 3. The $(GalNAc)_3$ conjugating molecule can be covalently conjugated to the 3'-end of the siRNA sense strand by starting the cycles with the solid phase support linked to the $(GalNAc)_3$ conjugating molecule.

Cleavage and deprotection: the sense strand S and the antisense strand AS of $(GalNAc)_3$-X2M2 were respectively added into concentrated ammonia and reacted at 55 °C for 16 h to complete the cleavage of the nucleic acid strand from the solid phase support and remove the protection groups on the base as well as the protection groups on the phosphoric acid. After the supernatant of ammonia was pipetted out, it was vacuum concentrated to dry, and then dissolved in DMSO with the addition of triethylamine trihydrofluoride for removing the protection of 2' TBDMS on ribose.

Purification and desalting: purification of the nucleic acid was accomplished with NaBr gradient elution by using a preparative ion chromatography purification column (Source 15Q). Products with qualified purity were collected and combined in tubes, and desalted by using reverse phase chromatography purification column.

Detection: the purity was measured by ion exchange chromatography (IEX-HPLC); and the molecular weight was analyzed by liquid chromatography-mass spectrometry (LC-MS).

Annealing: the S strand and the AS strand were mixed at an equimolar ratio and heated to 95 °C. After cooling at room temperature, the strands were allowed to form a duplex structure through hydrogen bond.

Identification: a Liquid Chromatography-Mass Spectrometry machine (LC-MS, purchased from Waters Co., model: LCT Premier) was used to perform molecular weight detection. The results are shown in FIG. 1a-1c, wherein it is found that the measured values are consistent with the theoretical values, demonstrating that the synthesized $(GalNAc)_3$-X2M2 is the designed target, that is, the double-stranded nucleic acid sequence carrying $(GalNAc)_3$ conjugating molecules.

**Test Example 1:** This test example was used to detect the toxicity of siRNA X2M2 of the present invention at cell level and animal level.

In accordance with conventional methods in the art, in HepG2.2.15 cells and rat primary hepatocytes, the cytotoxicity of siRNA X2M2 of the present invention was detected by MTT assay and no cytotoxicity was observed at 500 nM concentration (100 times higher than the efficacy concentration); in C57BL/6J mice, RBP131/X2M2 was intravenously administrated with a single dose of 10 mg/kg (33 times higher than the effective dose) and no animal death occurred and no clinical symptoms associated with adverse drug responses were observed during a period of continuous observation for 14 days; and in C57BL/6J mice, $(GalNAc)_3$-X2M2 conjugate was subcutaneously administrated with a single dose of 10 mg/kg and no animal death occurred and no clinical symptoms associated with adverse drug responses were observed during a period of continuous observation for 14 days, all the results indicating that siRNA X2M2 and the pharmaceutical composition or conjugate thereof according to the present invention have a relatively low cytotoxicity and high safety, showing the potential of clinical application.

**Experimental Example 1:** This experimental example was used to detect the inhibitory efficiency *in vitro* of the siRNAs obtained in Preparation Example 1 on the expression amount of HBV mRNA.

The siRNAs obtained in Preparation Example 1 were used to transfect HepG2.2.15 cells *in vitro.* Each group underwent 3 parallel tests and the experiment repeated at least 3 times. The final concentrations of the siRNAs were 50 nM, 10 nM and 1 nM, respectively. The expression levels of HBV mRNA in the above harvested cells were measured by real-time fluorescent qPCR 48 hours after transfection. Specifically, RNeasy Mini Kit (QIAGEN Co., Cat. 74106) was used to extract total RNAs according to the instructions thereof, and the extracted total RNAs were reverse transcribed

into cDNAs, and then the inhibitory efficiency of the siRNAs on the expression of HBV mRNA in HepG2.2.15 cells was detected by fluorescent qPCR method.

In this fluorescent qPCR method, GAPDH gene was used as the internal control gene, and the primers aiming at HBV and the primers aiming at GAPDH were used to detect HBV and GAPDH, respectively. Sequences of the primers are shown in Table 5 below.

Table 5 Sequences of the detection primers

| Gene | Upstream primer | Downstream primer |
|---|---|---|
| HBV | 5'-CCGTCTGTGCCTTCTCATCT-3' (SEQ ID NO: 6) | 5'-TAATCTCCTCCCCCAACTCC-3' (SEQ ID NO: 7) |
| GAPDH | 5'-AGAAGGCTGGGGCTCATTTG-3' (SEQ ID NO: 8) | 5'-AGGGGCCATCCACAGTCTTC-3' (SEQ ID NO: 9) |

In this fluorescent qPCR method, the inhibitory activity of siRNA was expressed as the remaining expression of HBV gene, and was calculated as follows:

The remaining expression of HBV gene = (the copy number of HBV gene in the test group / the copy number of GAPDH in the test group) / (the copy number of HBV gene in the mock group / the copy number of GAPDH in the mock group) X 100%.

Therein, each of the test groups was a group of the HepG2.2.15 cells treated with the siRNAs listed in Table 1, respectively, wherein said siRNAs included the siRNAs specifically targeting the X gene region of HBV and three control NC siRNAs; and the mock group was a group of the HepG2.2.15 cells without treatment of any siRNAs.

FIG. 2 shows the detection results of the inhibitory activity of the test siRNAs and control siRNAs listed in Table 1 on HBV mRNA expression in HepG2.2.15 cells. It can be seen from the results in FIG. 2 that, the inhibitory activity of each of the test siRNAs on HBV mRNA is similar to that of the naked sequence siRNA CA1, except for siRNA 3d and siRNA CA2, wherein, it can be seen from Table 1 that, siRNA 3d is a siRNA omitting the modification at the third nucleotide of the 5'-end of the siRNA A4 antisense strand and siRNA CA2 is a siRNA with alternating modifications of the nucleotides of the naked sequence siRNA CA1.

The results indicate that different chemical modifications to the siRNAs do affect the activity of the siRNAs, and siRNA X2M2 of the present invention exhibits an excellent activity at cell level.

**Experimental Example 2:** This experimental example was used to detect the stability of the siRNAs obtained in Preparation Example 1 in human plasma *in vitro.*

The siRNAs (20 μM, 10 μl) obtained in Preparation Example 1 were incubated with 50% human plasma (HP) at 37 °C *in vitro* for a certain time and samples were taken therefrom. Specifically, 10 μl samples were taken at 0, 2, 4, 6, 8, 24 and 48 hours respectively and immediately snap frozen in liquid nitrogen, followed by cryopreservation at -80 °C for future use. 20 wt% non-denaturing polyacrylamide gel was formulated, and 10 μl 5-fold diluted above samples (dilution solution: 1 x PBS buffer at pH 7.4) and 4 μl loading buffer (20 mM EDTA, 36 wt% glycerol, 0.06 wt% bromophenol blue) were mixed and then loaded into the gel to perform electrophoresis for about 60 minutes under 80 mA constant current. After electrophoresis finished, the gel was stained with 1 X Sybr Gold dye (Invitrogen, Cat. 11494) for 15 minutes followed by imaging. The results are shown in FIG. 3.

FIG. 3 shows the detection results of the stability of the test siRNAs and control siRNAs listed in Table 1 in human plasma *in vitro,* wherein the markers at two sides represent an equal amount of siRNA without the treatment of human plasma, which is used to indicate the size change of the treated siRNA. The ratio of the longest fragments remained after incubating the test siRNAs and control siRNAs with human plasma to the longest fragments of the siRNAs without the treatment of human plasma (i.e., the markers shown in FIG. 3) (also referred to as ratio of the longest fragments, RL) is calculated by reading the grayscale values quantitatively. The results are shown in Table 6.

Table 6 Quantitative results of siRNA plasma stability, with the concentration of human plasma being 50%

| Sample | RL | | | | | | |
|---|---|---|---|---|---|---|---|
| | 0h | 2h | 4h | 6h | 8h | 24h | 48h |
| NC | 100.0% | 83.9% | 69.7% | 58.4% | 52.6% | 36.2% | 26.0% |

(continued)

| Sample | RL | | | | | | |
|---|---|---|---|---|---|---|---|
| | 0h | 2h | 4h | 6h | 8h | 24h | 48h |
| NC-pos | 100.0% | 93.4% | 95.9% | 84.9% | 88.6% | 87.1% | 90.0% |
| NC-bas | 100.0% | 95.1% | 89.8% | 86.8% | 85.3% | 91.1% | 90.8% |
| CA1 | 100.0% | 0% | 0% | 0% | 0% | 0% | 0% |
| CA2 | 100.0% | 86.9% | 76.5% | 68.8% | 67.7% | 48.7% | 52.0% |
| A4 | 100.0% | 97.5% | 93.7% | 106.0% | 91.7% | 90.0% | 81.2% |
| 1d | 100.0% | 90.0% | 97.2% | 93.3% | 87.5% | 55.7% | 65.1% |
| 2d | 100.0% | 97.6% | 88.2% | 82.2% | 84.5% | 82.1% | 78.4% |
| 3d | 100.0% | 96.6% | 99.6% | 93.3% | 87.1% | 75.0% | 41.6% |
| 8d | 100.0% | 78.2% | 72.7% | 59.7% | 48.3% | 34.0% | 25.9% |
| 14d | 100.0% | 94.6% | 90.0% | 85.6% | 86.0% | 78.0% | 84.6% |
| X2M2(S++) | 100.0% | 101.5% | 98.2% | 98.6% | 93.0% | 92.1% | 90.9% |
| X2M2(S--) | 100.0% | 111.3% | 97.5% | 90.2% | 96.1% | 109.1% | 93.9% |

The results show that, the unmodified naked siRNA CA1 degrades rapidly immediately after the addition of human plasma, showing a very poor stability in plasma; while all the modified siRNAs are able to improve plasma stability to a certain extent, wherein the plasma stability of siRNA CA2 with up to 20 chemical modifications is improved but is still not satisfactory. On the other hand, whether or not the phosphodiester bonds between the overhang dTdT at the 3'-end are replaced by phosphorothioate diester bonds, siRNA X2M2 of the present invention exhibits more excellent plasma stability than siRNA A4, and can ensure an RL of 90% or higher even after incubation with human plasma for 48 hours. Considering that siRNA X2M2 of the present invention even omits one nucleotide modification on the basis of siRNA A4, these results are very surprising. Furthermore, it is more surprising that, as can be seen from FIG. 3, although siRNA A4 is able to maintain an RL of 81.2% after incubation with human plasma for 48 h, it can be observed that the band thereof significantly shifts downward gradually (similar situations also present in other test siRNAs specifically targeting the X gene region of HBV), that is to say, a certain degree of degradation still occurs (maybe due to the cleavage of its terminal base(s) by RNase). However, siRNA X2M2 of the present invention is able to maintain a full-length state all the time.

**Experimental Example 3:** This experimental example was used to detect the inhibitory efficiency of the RBP131/siRNA liposome formulation obtained in Preparation Example 2 on the expression amount of HBV mRNA in HBV transgenic mice C57BL/6J-Tg (Alb1HBV)44Bri/J (purchased from Beijing Vital River Laboratory Animal Technology Co., Ltd.).

Therein, the test siRNAs used included siRNAs X2M2(S++), A4, 3d, X2M2(S--) listed in Table 1, and an unrelated siRNA siFL867 specifically against Firefly Luciferase was used as negative control, the sequences thereof were as follows:

Sense strand: 5'-CCCUAUUCUCCUUCUUCGCdTdT-3' (SEQ ID NO: 10);
Antisense strand: 5'-GCGAAGAAGGAGAAUAGGGdTdT-3' (SEQ ID NO: 11)

At first, 24 C57BL/6J-Tg(Alb1HBV)44Bri/J mice were randomly divided into 6 groups (4 mice in each group, all female) based on HBsAg content in serum, PBS control group, RBP131/siFL867 group, RBP131/X2M2(S++) group, RBP131/A4 group, RBP131/3d group and RBP131/X2M2(S--) group, respectively. The drug dosages for all animals were calculated according to the body weight. A single dose was administered via tail vein, with the administration dose being 1 mg/kg and the volume being10 ml/kg. Animals were sacrificed on day 3 after administration, and were subjected to gross anatomy to observe whether the organs in the body were diseased. The diseased tissues by visual observation were kept in 10% formalin for further pathological observation. The liver was collected and kept with RNA later (Sigma Aldrich), the liver tissue was homogenized with tissue homogenizer, and then total RNAs were extracted and obtained by using Trizol according to the standard procedure for total RNA extraction.

The expression level of HBV mRNA in liver tissue was detected by real-time fluorescent qPCR. Specifically, ImProm-II™ reverse transcription kit (Promega) was used to reverse transcribe the extracted total RNAs into cDNAs

according to the instruction thereof, and then the fluorescent qPCR kit (Beijing Cowin Biosicences Co., Ltd) was used to detect the inhibitory efficiency of siRNA on the expression of HBV mRNA in liver tissue. In this fluorescent qPCR method, β-actin gene was used as the internal control gene, and the primers aiming at HBV and the primers aiming at β-actin were used to detect HBV and β-actin, respectively.

The sequences of the detection primers are shown in Table 7.

Table 7 Sequences of the detection primers

| Gene | Upstream primer | Downstream primer |
|---|---|---|
| HBV | 5'-CCGTCTGTGCCTTCTCA TCT -3' | 5'-TAATCTCCTCCCCCAACTCC-3' |
| β-actin | 5'-AGCTTCTTTGCAGCTCCTTCGT TG-3' (SEQ ID NO: 12) | 5'-TTCTGACCCATTCCCACCATCACA-3' (SEQ ID NO: 13) |

In this fluorescent qPCR method, the inhibitory activity of siRNA was expressed as the remaining expression of HBV gene, and was calculated as follows:
The remaining expression of HBV gene = (the copy number of HBV gene in the test group / the copy number of β-actin in the test group) / (the copy number of HBV gene in the control group / the copy number of β-actin in the control group) X 100%.

Therein, the control group was a group of control mice administrated with PBS in this experiment and each of the test groups was a group of mice administrated with different drug compositions, respectively. The results are shown in FIG. 4.

The results show that, the inhibitory activities of RBP131/X2M2(S++) and RBP131/ X2M2(S--) on the mRNA of the X gene region of HBV gene in the liver tissue of hepatitis B mice are comparable, both of which are very satisfactory with an inhibition rate being around 90%; the negative control RBP131/siFL867 and RBP131/3d in the test group have no inhibitory effects on the mRNA of the X gene region of HBV in the liver tissue of hepatitis B mice; and the inhibition rate of RBP131/A4 on the mRNA of the X gene region of HBV in the liver tissue of hepatitis B mice is about 70%. These results demonstrate that, at animal level, under the circumstance that the sequences of the siRNAs themselves are identical, irrational modification strategies may even result in complete loss of siRNA activity, for example, the activity exhibited by RBP131/3d; on the contrary, siRNA X2M2 of the present invention has a quite excellent biological activity in the disease model mice, which is even higher than that of siRNA A4 in the granted prior application CN102140461B of the applicant.

In addition, the RBP130/X2M2(S++) and RBP130/X2M2(S--) liposome formulations obtained in Preparation Example 2 were tested according to the same manner. These liposome formulations exhibited similar effects to those of RBP131/X2M2(S++) and RBP131/X2M2(S--).

**Experimental Example 4:** This experimental example was used to detect the inhibitory efficiency of different doses of X2M2 encapsulated by RBP131 on the expression amount of serum HBsAg in HBV transgenic mice at different time points.

RBP131/siRNA liposome formulation was prepared according to the method of Preparation Example 2. 80 HBV transgenic mice (named M-TgHBV, purchased from the Animal Department of Shanghai Public Health Clinical Center, and the method for preparing the transgenic mice was described in Ren J., et al., J. Medical Virology. 2006, 78: 551-560) were randomly divided into 8 groups (10 mice in each group, 4 male and 6 female) based on HBsAg content in serum, 1 X PBS control group, RBP131/NC control group (1 mg/kg), IFN interferon control group (25 ng/g), three sample groups of different doses of RBP131/X2M2(S++) (0.1 mg/kg, 0.3 mg/kg and 1 mg/kg, respectively), sample groups of a single dose of RBP131/X2M2(S--) (1 mg/kg) and a single dose of RBP131/A4 (1 mg/kg), respectively. The drug dosages for all animals were calculated based on the body weight and the administration volume was 10 ml/kg. The PBS group and each of the RBP131/siRNA groups were administered with a single dose via tail vein, and the interferon group was administered subcutaneously twice a week, i.e., administrated at day 0, 4, 7, 11 and 14, 5 times in total. The blood was taken from mouse orbital venous plexus before administration and at day 7, 14, 21, and 28 after administration, and serum HBsAg levels were measured for each time point.

The volume of the blood taken out from the orbit was about 0.5 ml each time, and the volume of the serum was no less than 200 μl after centrifugation. HBsAg CLIA kit (Autobio, CL0310) was used to detect the expression levels of HBsAg in serum. The remaining expression of HBsAg was calculated as follows:
The remaining expression of HBsAg = (the content of HBsAg in the test group / the content of HBsAg in the PBS control group) X 100%, wherein the content of HBsAg was expressed in nanograms of HBsAg per gram of liver tissue proteins.

The results are shown in FIG. 5. It can be seen that, both IFN interferon control group and RBP131/NC control group have no inhibitory effect on the expression of HBsAg in mouse serum at different time points after administration.

RBP131/X2M2 and RBP131/A4 inhibit the expression of HBsAg in serum in a dose-dependent manner. Therein, regarding RBP131/X2M2(S++), the inhibition rate of low dose (0.1 mg/kg) on the 7th day is 68%, the inhibitory effect of middle dose (0.3 mg/kg) can be maintained for more than 14 days, and the inhibitory effect of high dose (1 mg/kg) can be maintained for more than 28 days; and in the case of equal dose (1 mg/kg), the inhibitory effects of RBP131/X2M2(S++) and RBP131/X2M2(S--) on the expression of HBsAg in serum are always higher than that of RBP131/A4. It can be seen that the biological activity of siRNA X2M2 of the present invention is superior to siRNA A4 in animals, further enhancing the clinical application potential thereof.

On the other hand, this experiment also shows that RBP131/X2M2 of the present invention has a significant inhibitory effect on HBsAg compared to interferon, a traditional anti-hepatitis B drug, demonstrating that siRNA has the possibility of functionally curing viral hepatitis B.

In addition, the RBP130/X2M2(S++) and RBP130/X2M2(S--) liposome formulations obtained in Preparation Example 2 were tested according to the same manner. These liposome formulations exhibited similar effects to those of RBP131/X2M2(S++) and RBP131/X2M2(S--).

**Experimental Example 5:** This experimental example was used to detect the inhibitory efficiency of different doses of X2M2 encapsulated by RBP131 on the expression amount of hepatic HBsAg in HBV transfected mice based on high-pressure injection at different time points.

The RBP131/X2M2 complex was prepared according to the method in Preparation Example 2. 72 C3H/HeN mice injected with HBV plasmid by high-pressure injection (named pHBV, purchased from the Animal Department of Shanghai Public Health Clinical Center, and the methods for preparing the high-pressure injected mice were described in Peng XH., et al., World J. Gastroenterol. 2015, 21(12):3527-3536) were randomly divided into 6 groups (12 mice in each group, half female and half male) based on HBsAg content in serum, 1 X PBS control group, ETV entecavir control group (0.1 mg/kg), three sample groups of different doses of RBP131/X2M2(S++) (0.3 mg/kg, 1 mg/kg and 3 mg/kg, respectively) and one sample group of a single dose of RBP131/X2M2(S--) (1 mg/kg), respectively. The drug dosages for all animals were calculated based on the body weight and the administration volume was 10 ml/kg. The PBS group and each of the RBP131/X2M2 groups were administered with a single dose via tail vein, and the entecavir group was administered by gastric gavage daily for 7 consecutive days, i.e., once a day from day 0 to day 6. Half of the animals was sacrificed in batches at day 7 and day 28, respectively. The expression levels of HBsAg in liver tissue were detected by the detection method as described above.

The results are shown in FIG. 6. It can be seen that, RBP131/X2M2(S++) with the dose of 0.3 mg/kg shows a sign of inhibition on the expression levels of HBsAg in the liver, while RBP131/X2M2(S++) with the doses of 1 mg/kg and 3 mg/kg can significantly inhibit the expression levels of HBsAg in the liver, with the inhibition rate of HBsAg in the liver being up to 90% or higher compared to that of the PBS group at day 7 after administration, whereas entecavir, a current mainstream anti-hepatitis B nucleoside drug, has no inhibitory effect on the expression of HBsAg in the liver. At the same time, the inhibitory efficiencies of RBP131/X2M2(S--) on the expression amount of hepatic HBsAg in high-pressure injected hepatitis B mice pHBV at different time points are similar to those of RBP131/X2M2(S++), suggesting the potential advantage of RBP131/X2M2 for functionally curing viral hepatitis B.

In addition, the RBP130/X2M2(S++) and RBP130/X2M2(S--) liposome formulations obtained in Preparation Example 2 were tested according to the same manner. These liposome formulations exhibited similar effects to those of RBP131/X2M2(S++) and RBP131/X2M2(S--).

**Experimental Example 6:** This experimental example was used to detect the inhibitory efficiency of commercially available carriers for delivering siRNA *in vivo* manufactured by different manufacturers, including Invivo fectamine 2.0 (purchased from Life technology), invivo jetPEI (purchased from PolyPlus-transfection(PT)) and Entranster (purchased from Engreen Biosystem), as well as RBP131 of the present invention, on the expression amount of HBV mRNA in HBV transgenic model mice C57BL/6J-Tg (Alb1HBV)44Bri/J (purchased from Beijing Vital River Laboratory Animal Technology Co., Ltd.) after they encapsulated and carried siRNA X2M2.

The corresponding carrier/siRNA compositions were prepared by encapsulating siRNA X2M2(S++) of the present invention into the above commercial carriers according to the standard operating procedures provided by the manufacturers, labeled as IVF2.0/X2M2, invivo jetPEI/X2M2 and Entranster/X2M2, respectively. Meanwhile, the RBP131/X2M2 composition was prepared according to the method of Preparation Example 2.

30 C57BL/6J-Tg(Alb1HBV)44Bri/J mice were randomly divided into 5 groups (6 mice in each group, half male and half female) based on HBsAg content in serum, PBS control group, sample groups of IVF2.0/X2M2, invivo jetPEI/X2M2, Entranster/X2M2, and RBP131/X2M2, respectively. The drug dosages for all animals were calculated based on the body weight and the animals were administered with a single dose via tail vein. The administration dose of the three groups of IVF2.0/X2M2, invivo jetPEI/X2M2 and Entranster/X2M2 was 2.5 mg/kg (siRNA), and that of RBP131/X2M2 group was 0.5 mg/kg (siRNA). The administration volume of all 5 groups was 10 ml/kg. Animals were sacrificed on day 3 after administration, and were subjected to gross anatomy to observe whether the organs in the body were diseased. The diseased tissues by visual observation were kept in 10% formalin for further pathological observation. The liver was collected and kept with RNA later (Sigma Aldrich); the liver tissue was homogenized with tissue homogenizer, and then total RNAs were extracted and obtained by using Trizol according to the standard operating procedures for total RNA extraction.

Real-time fluorescent qPCR was used to detect the expression levels of HBV mRNA in the liver tissue. The detection method was the same as that described in Experimental Example 3 and the detection primers were the same as those described in Table 5.

In this fluorescent qPCR method, the inhibitory activity of siRNA was calculated as follows:

The inhibitory activity of siRNA = [1- (the copy number of HBV gene in the test group/ the copy number of GAPDH in the test group) / (the copy number of HBV gene in the control group / the copy number of GAPDH in the control group)] X 100%.

Therein, the control group was a group of control mice administered with PBS in this experiment and each of the treatment groups was a group of mice administered with different drug compositions, respectively.

Table 8 shows the detection results of the inhibitory activity of each group of test samples on the expression of HBV mRNA in C57BL/6J-Tg(Alb1HBV)44Bri/J transgenic mice.

Table 8 Inhibitory activity on HBV mRNA expression

| Group of test samples | Source of carriers (manufactures) | siRNA dose | Administration frequency | Treatment time | Experimental animals and numbers | mRNA inhibitory efficiency |
|---|---|---|---|---|---|---|
| 1×PBS | Self-prepared | 0 mg/kg | Single dose | 72 h | Bri44, 22-27g, 3♀+3♂ | 0% |
| *Invivo* fectamine 2.0 (IVF2.0)/ X2M2 | Life Technologies | 2.5 mg/kg | Single dose | 72 h | Bri44, 22-27g, 3♀+3♂ | 61% |
| invivo jetPEI/X2 M2 | PolyPlus-trans fection(PT) | 2.5 mg/kg | Single dose | 72 h | Bri44, 22-27g, 3♀+3♂ | 49% |
| Entranster /X2M2 | Engreen Biosystem | 2.5 mg/kg | Single dose | 72 h | Bri44, 22-27g, 3♀+3♂ | 10% |
| RBP131/ X2M2 | Suzhou Ribo | 0.5 mg/kg | Single dose | 72 h | Bri44, 22-27g, 3♀+3♂ | 95% |

The results show that the inhibition rate of RBP131/X2M2 group on HBV mRNA in hepatitis B model mice increases by 34% even when compared with IVF2.0/X2M2 group, the one having the best effect among the pharmaceutical compositions formed by other commercial carriers; in addition, the inhibitory activity of RBP131/X2M2 group is nearly 2 times the activity of invivo jetPEI/X2M2 group and is nearly 10 times the activity of Entranster/X2M2 group. Furthermore, considering that the administration dose of RBP131/X2M2 group is only 1/5 of the other three groups, RBP131/X2M2 shows an extremely high biological activity compared with the pharmaceutical compositions formed by other commercial carriers.

**Experimental Example 7:** This experimental example was used to detect the inhibitory efficiency of carriers for delivering siRNA *in vivo* from different literature sources and RBP131 of the present invention on the expression amount of ApoB mRNA (or blood lipid) in normal C57BL/6J mice after they encapsulated and carried the siRNA against apolipoprotein B (ApoB) gene (siApoB: sense strand (5'→3') GUCAUCACACUGAAUAC CAAUdTdT (SEQ ID NO: 16); antisense strand (5'→3') AUUGGUAUUCAGUGUGAUGA CACdTdT (SEQ ID NO: 17)) to form liposome formulations. The main function of ApoB is to transport the lipid in the liver into the blood circulation system. When the expression of ApoB is inhibited, accordingly, the lipid in the liver will accumulate and the lipid level in the blood will decrease.

According to the descriptions in the literatures (Biomaterials: 2012 Jun; 33 (18): 4653-64 and Biomaterials. 2013 Apr; 34 (12): 3120-31), the carriers and the siRNAs were mixed and the corresponding liposome formulations were prepared. Specifically: (1) Preparation of binary and ternary complexes: based on the desired charge ratio of the complex (in the charge ratio of the binary and ternary complex (N/P, N/P/C), N represents the molar amount of amino groups in the PCL-g-PDMAEMA molecule, P represents the molar amount of the phosphate groups of the siRNA molecule, and C represents the molar amount of carboxyl groups in the PGA-g-PEG molecule), the siRNA (containing the required

amount of siRNA) and PCL-g-PDMAEMA nanoparticles (containing the desired amount of amino groups) were mixed and incubated at room temperature for 15 minutes to obtain a binary complex. A certain amount of PGA-g-PEG (containing the desired amount of carboxyl groups) was added to the binary complex and continued to be incubated at room temperature for 15 minutes to obtain a ternary complex. (2) Preparation of PEA and PEAG complexes: The N/P ratios of polymers PEA and PEAG to the siRNAs were calculated based on the number of moles of amino/guanidine (N) on the polymer and the number of moles of phosphoric acid (P) of the siRNA. When preparing the complex, a certain amount of polymer (containing the desired amount of amino/guanidine) was mixed with a certain amount of the siRNA solution (containing the desired amount of siRNA) by a pipette and incubated at room temperature for 20 minutes to form the final PEA/siRNA and PEAG/siRNA mixtures. For RBP131, the RBP131/siApoB complex was prepared according to the method of Preparation Example 2. The volume of these complexes can be adjusted with 1 x PBS according to the needs prior to administration to suit the requirements of the corresponding experiments.

36 normal C57BL/6J mice were randomly divided into 6 groups (6 mice in each group, half female and half male), PBS control group, Binary Complex 5: 1 (binary complex) group, Ternary Complex 5: 1: 2 (Ternary complex) group, PEA/siApoB 10: 1 group, PEAG/siApoB 10: 1 group, and RBP131/siApoB group, respectively. The drug dosages for all animals were calculated based on the body weight and the animals were administered with a single dose via tail vein. The administration dose of Binary Complex 5: 1 (binary complex) group, Ternary Complex 5: 1: 2 (ternary complex) group, PEA/siApoB 10: 1 group and PEAG/ siApoB 10: 1 group was 2.5 mg/kg (siRNA), and that of RBP131/siApoB was 0.5 mg/kg (siRNA). The administration volume of all 6 groups was 10 ml/kg. Animals were sacrificed on day 3 after administration. The blood was taken out from mouse orbital venous plexus and centrifuged to obtain serum; and the animals were subjected to gross anatomy to observe whether the organs in the body were diseased. The diseased tissues by visual observation were kept in 10% formalin for further pathological observation. The liver was collected and kept with RNA later (Sigma Aldrich); the liver tissues were homogenized with tissue homogenizer, and then total RNAs were extracted and obtained by using Trizol according to the standard operating procedures for total RNA extraction.

The expression level of ApoB mRNA in liver tissue was detected by real-time fluorescent qPCR. Specifically, ImProm-II™ reverse transcription kit (Promega) was used to reverse transcribe the extracted total RNAs into cDNAs according to the instruction thereof, and then the fluorescent qPCR kit (Beijing Cowin Biosicences Co., Ltd) was used to detect the inhibitory efficiency of siRNA on the expression of ApoB mRNA in liver tissue.

Table 9 Sequences of the detection primers

| Gene | Upstream primer | Downstream primer |
|---|---|---|
| ApoB | 5'-TTCCAGCCATGGGCAACTTTACCT-3' (SEQ ID NO: 14) | 5'-TACTGCAGGGCGTCAGTGACAAAT-3' (SEQ ID NO: 15) |
| β-actin | 5'-AGCTTCTTTGCAGCTCCTTCGTTG-3' | 5'-TTCTGACCCATTCCCACCATCACA-3' |

In this fluorescent qPCR method, the inhibitory activity of siRNA was calculated as follows:

The inhibitory activity of siRNA = [1- (the copy number of ApoB gene in the test group / the copy number of β-actin in the test group) / (the copy number of ApoB gene in the control group / the copy number of β-actin in the control group)] X 100%.

Therein, the control group was a group of control mice administered with PBS in this experiment and each of the test groups was a group of mice administered with different drug compositions, respectively.

The level of blood lipid in serum was directly measured with PM4000/3 serum biochemical analyzer (SABA, Italy).

Table 10 shows the detection results of the inhibitory activity of each group of test samples on the expression of ApoB mRNA and the blood lipid expression levels in C57BL/6J normal mice.

Table 10 Inhibitory activity on ApoB mRNA expression and the blood lipid expression level

| Group of test samples | Source of carriers (manufactures or literature sources) | siRNA dose | Administration frequency | Treatment time | Experimental animals | mRNA inhibitory efficiency | Reduction of blood lipid |
|---|---|---|---|---|---|---|---|
| 1× PBS | Self-prepared | 0 mg/kg | Single dose | 72 h | C57BL/6J, 18-22g, 3♀+3♂ | 0% | 0% |
| Binary Complex 5:1 (binary carrier) | Biomaterials. 2012 Jun;33(18):46 53-64. | 2.5 mg/kg | Single dose | 72 h | C57BL/6J, 18-22g, 3 3♀+3♂ | 38% | 33% |
| Ternary Complex 5:1:2 (ternary carrier) | | 2.5 mg/kg | Single dose | 72 h | C57BL/6J, 18-22g, 3♀+3♂ | 12% | 8% |
| PEA/siAp oB 10:1 | Biomaterials. 2013 Apr;34(12):3 120-31. | 2.5 mg/kg | Single dose | 72 h | C57BL/6J, 18-22g, 3♀+3♂ | 20% | 16% |
| PEAG/siA poB 10:1 | | 2.5 mg/kg | Single dose | 72 h | C57BL/6J, 18-22g, 3♀+3♂ | 40% | 34% |
| RBP131/si ApoB | Suzhou Ribo | 0.5 mg/kg | Single dose | 72 h | C57BL/6J, 18-22g, 3♀+3♂ | 95% | 93% |

The results show that the inhibitory effect of RBP131/siApoB group on ApoB mRNA and blood lipid is significantly better than that of other pharmaceutical compositions, wherein the inhibition rate of RBP131/siApoB on blood lipid is 2.8 times the rate of Binary Complex 5: 1 (binary complex) group and PEAG/siApoB 10: 1 group and 11.6 times the rate of Ternary Complex 5: 1: 2 (ternary complex) group; moreover, the administration dose of RBP131/siApoB group still is only 1/5 of the other four groups. Therefore, RBP131/siApoB shows an extremely high bioactivity compared to the pharmaceutical compositions formed by other carriers.

**Experimental Example 8:** This experimental example was used to detect the inhibitory efficiency of different doses of $(GalNAc)_3$-X2M2 conjugate on the expression amounts of HBV mRNA in liver and HBsAg in serum in HBV transfected mice based on high-pressure injection.

According to the method of Preparation Example 3, $(GalNAc)_3$-X2M2 conjugate and $(GalNAc)_3$-NC conjugate were prepared ($(GalNAc)_3$-NC conjugate was prepared in the same manner as that of $(GalNAc)_3$-X2M2 conjugate, with the only exception that the sequences of the siRNA were replaced with those of NC listed in Table 1). 60 C3H/HeN mice injected with HBV plasmid by high-pressure injection (named pHBV, purchased from the Animal Department of Shanghai Public Health Clinical Center, and the methods for preparing the high-pressure injected mice were described in Peng XH., et al., World J. Gastroenterol. 2015, 21(12):3527-3536) were randomly divided into 6 groups (10 mice in each group, half female and half male) based on HBsAg content in serum, 1 X PBS control group, ETV entecavir control group (0.1 mg/kg), $(GalNAc)_3$-NC control group (3 mg/kg), and three sample groups of different doses of $(GalNAc)_3$-X2M2 (0.3 mg/kg, 1 mg/kg and 3 mg/kg, respectively), respectively. The drug dosages for all animals were calculated based on the body weight and the administration volume was 10 ml/kg. The PBS group, $(GalNAc)_3$-NC group and each of the $(GalNAc)_3$- X2M2 groups were administered with a single dose subcutaneously, and the entecavir group was administered by gastric gavage daily for 14 consecutive days, i.e., once a day from day 0 to day 13. All animals were sacrificed at day 14 to detect the expression levels of HBV mRNA in liver and HBsAg in serum. The detection methods were the same as those in Experimental Example 3 and Experimental Example 4, respectively.

The results are shown in FIG. 7 and FIG. 8. It can be seen that, after single subcutaneous administration, both ETV entecavir control group and $(GalNAc)_3$-NC control group have no inhibitory effect on the expression of HBV mRNA in liver and HBsAg in serum in mice. $(GalNAc)_3$-X2M2 reduces the contents of HBV mRNA in liver and HBsAg in serum in HBV mice in a dose-dependent way, and the inhibition rates are shown in Table 11 below.

Table 11 Inhibition on the expression of HBV mRNA in liver and HBsAg in serum in mice

| Group | | Inhibition rates of HBV X mRNA in liver (%) | Inhibition rates of HBsAg in serum (%) |
|---|---|---|---|
| ETV | 0.1 mg/kg | -5.0 | 2.8 |
| $(GalNAc)_3$-NC | 3 mg/kg | -5.8 | -0.4 |
| $(GalNAc)_3$-X2M2 | 0.3 mg/kg | 45.8 | 43.6 |
| | 1 mg/kg | 90.6 | 87.0 |
| | 3 mg/kg | 96.9 | 95.6 |

It can be seen that, in animal bodies, the $(GalNAc)_3$-X2M2 conjugate of the present invention has a very good biological activity, having the potential for clinical application. In addition, the $(GalNAc)_3$-X2M2 of the present invention has a significant inhibitory effect on HBsAg compared to entecavir, which is a clinical first-line anti-hepatitis B drug, further highlighting the possibility of $(GalNAc)_3$-siRNA conjugate for functionally curing viral hepatitis B.

**Experimental Example 9:** This experimental example was used to detect the inhibitory efficiency of different doses of $(GalNAc)_3$-X2M2 conjugate on the expression amount of HBsAg in serum in HBV transfected mice based on high-pressure injection at different time points.

The $(GalNAc)_3$-X2M2 conjugate was prepared according to the method of Preparation Example 3. 40 C3H/HeN mice injected with HBV plasmid by high-pressure injection (named pHBV, purchased from the Animal Department of Shanghai Public Health Clinical Center, and the methods for preparing the high-pressure injected mice were described in Peng XH., et al., World J. Gastroenterol. 2015, 21(12):3527-3536) were randomly divided into 4 groups (10 mice in each group, half female and half male) based on HBsAg content in serum, 1 X PBS control group and three sample groups of different doses of $(GalNAc)_3$-X2M2 (0.3 mg/kg, 1 mg/kg and 3 mg/kg, respectively), respectively. The drug doses for all animals were calculated based on the body weight and the administration volume was 10 ml/kg. The PBS group and each of the $(GalNAc)_3$-X2M2 groups were administered with a single dose subcutaneously and the HBsAg content in serum in animals was measured weekly. All animals were sacrificed at day 56 after administration to detect the expression levels of HBsAg in serum by the method as described above.

The results are shown in FIG. 9. It can be seen that, $(GalNAc)_3$-X2M2 reduces the contents of HBsAg in serum in HBV mice in a dose-dependent manner at different time points after administration. The inhibitory effect of a single administration of 0.3 mg/kg $(GalNAc)_3$-X2M2 on serum HBsAg can be maintained to day 42, and the inhibitory effects of a single administration of 1 mg/kg and a single administration of 3 mg/kg $(GalNAc)_3$-X2M2 on serum HBsAg can be maintained to day 49. The inhibitory effect of a single administration of 1 mg/kg $(GalNAc)_3$-X2M2 on HBsAg is more than 90% after 2 weeks. It can be seen that a single administration of $(GalNAc)_3$-X2M2 can effectively inhibit serum HBsAg in hepatitis B mice for a long period of time, suggesting that it will provide a potential possibility of functional cure and compliance improvement for patients with chronic hepatitis B.

## Claims

**1.** A siRNA with the sequences as follows:

Sense strand 5'-CmCmUmUmGAGGCmAUmACmUmUmCmAAA-dTdT-3'; Antisense strand 5'-UfUmUf-GAAGUfAUGCCUfCAAGG-dTdT-3',
wherein the uppercase letters C, G, U, A and T represent the base composition of the nucleotide; the lowercase letter d indicates that the one nucleotide on the right side of the letter d is a deoxyribonucleotide; the lowercase letter m indicates that the 2'-hydroxyl group of the ribose group of the one nucleotide on the left side of the letter m is substituted by a methoxy group; and the lowercase letter f indicates that the 2'-hydroxyl group of the ribose group of the one nucleotide on the left side of the letter f is substituted by fluorine.

**2.** The siRNA according to claim 1, wherein the phosphodiester bond between the overhang dTdT at the 3'-end of the sense strand and/or the antisense strand of the siRNA is replaced by phosphorothioate diester bond.

**3.** A pharmaceutical composition comprising the siRNA according to claim 1 and a pharmaceutically acceptable carrier.

**4.** The pharmaceutical composition according to claim 3, wherein the pharmaceutically acceptable carrier is a lipid mixture consisting of an amine-containing compound, a cholesterol, and a pegylated lipid, wherein the amine-containing compound is a compound represented by Formula I or a pharmaceutically acceptable salt thereof:

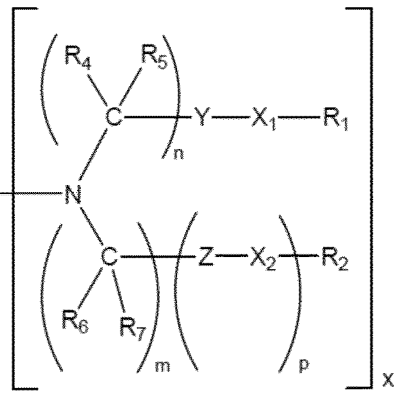
Formula I

wherein:

each of $X_1$ and $X_2$ is independently selected from O, S, N-A or C-A, wherein A is hydrogen or a $C_1$-$C_{20}$ hydrocarbon chain;
each of Y and Z is independently selected from C=O, C=S, S=O, CH-OH or $SO_2$;
each of $R_1$, $R_2$, $R_3$, $R_4$, $R_5$, $R_6$ and $R_7$ is independently selected from hydrogen; a cyclic or an acyclic, substituted or unsubstituted, branched or unbranched aliphatic group; a cyclic or an acyclic, substituted or unsubstituted, branched or unbranched heteroaliphatic group; a substituted or unsubstituted, branched or unbranched acyl group; a substituted or unsubstituted, branched or unbranched aryl group, a substituted or unsubstituted, branched or unbranched heteroaryl group;
x is an integer having the value between 1 and 10;
n is an integer having the value between 1 and 3, m is an integer having the value between 0 and 20, p is an integer having the value of 0 or 1, wherein if m=p=0, then $R_2$ is hydrogen,
with the further proviso that if at least one of n or m has the value of 2, then $R_3$ and nitrogen in Formula I form a structure as represented by Formula II or Formula III:

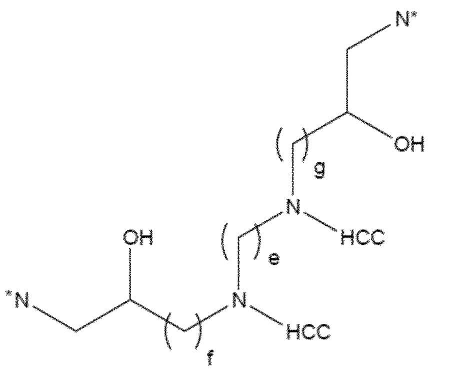
Formula II;

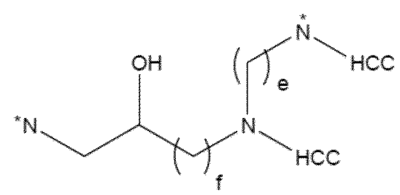
Formula III,

wherein each of g, e and f is independently an integer having the value between 1 and 6, "HCC" symbolizes a hydrocarbon chain, and each * indicates the nitrogen atom in Formula I.

**5.** The pharmaceutical composition according to claim 4, wherein
the amine-containing compound is amine-containing compound 72 or amine-containing compound 87 as follows:

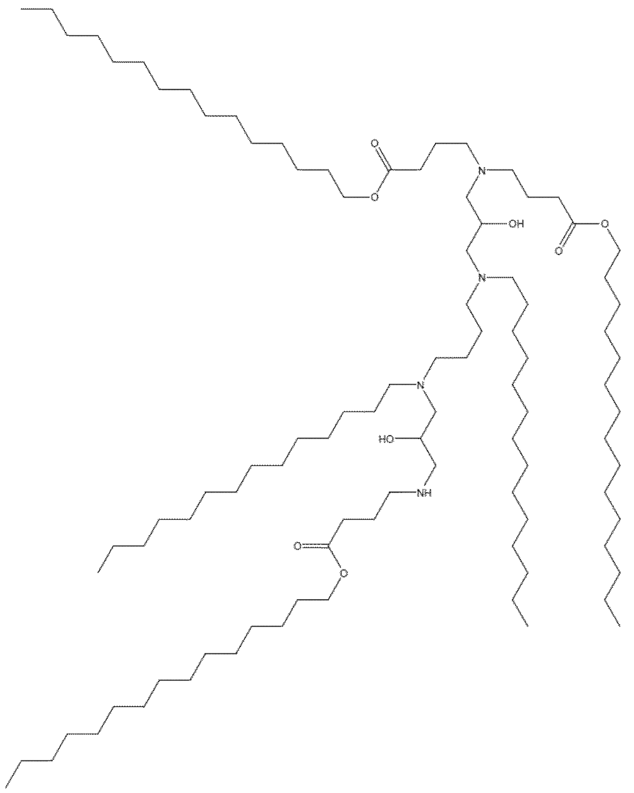

72 ;

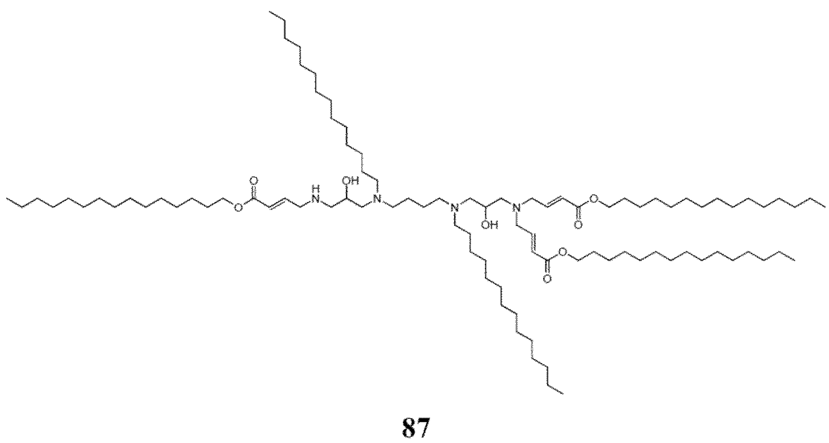

87 ,

and the pegylated lipid is 1,2-dipalmitoyl-sn-glycero-3-phosphoethanolamine-N-[methoxy(polyethylene glycol)-2000].

**6.** The pharmaceutical composition according to claim 4, wherein the molar percentages of the amine-containing compound, the cholesterol, and the pegylated lipid in the pharmaceutically acceptable carrier are 19.7%-80% for the amine-containing compound, 19.7%-80% for the cholesterol, and 0.3%-50% for the pegylated lipid,
preferably, the molar percentages of the amine-containing compound, the cholesterol, and the pegylated lipid in the pharmaceutically acceptable carrier are 50%-70% for the amine-containing compound, 20%-40% for the cholesterol, and 3%-20% for the pegylated lipid.

**7.** The pharmaceutical composition according to claim 5, wherein the molar percentages of amine-containing compound 87, cholesterol, and 1,2-dipalmitoyl-*sn*-glycero-3-phosphoethanolamine-N-[methoxy(polyethylene glycol)-2000] in the pharmaceutically acceptable carrier are 19.7%-80% for amine-containing compound 87, 19.7%-80% for cholesterol, and 0.3%-50% for 1,2-dipalmitoyl-sn-glycero-3-phosphoethanolamine-N-[methoxy(polyethylene glycol)-2000],
preferably, the molar percentages of amine-containing compound 87, cholesterol, and 1,2-dipalmitoyl-*sn*-glycero-3-phosphoethanolamine-N-[methoxy(polyethylene glycol)-2000] in the pharmaceutically acceptable carrier are 50%-70% for amine-containing compound 87, 20%-40% for cholesterol, and 3%-20% for 1,2-dipalmitoyl-*sn*-glycero-3-phosphoethanolamine-N-[methoxy(polyethylene glycol)-2000] .

**8.** The pharmaceutical composition according to claim 3, wherein the weight ratio of the siRNA to the pharmaceutically acceptable carrier is 1: (1-50).

**9.** A siRNA conjugate obtained by conjugating the siRNA according to claim 1 to a pharmaceutically acceptable conjugating molecule, wherein the conjugating molecule comprises a pharmaceutically acceptable targeting molecule and an optional linker.

**10.** The siRNA conjugate according to claim 9, wherein the pharmaceutically acceptable targeting molecule is a ligand for asialoglycoprotein receptor, preferably, the pharmaceutically acceptable targeting molecule is galactose or N-acetylgalactosamine.

**11.** The siRNA conjugate according to claim 10, wherein the galactose or N-acetylgalactosamine is covalently conjugated to the siRNA by the linker, preferably, the galactose or N-acetylgalactosamine is covalently conjugated to the siRNA at the 3'-end of the sense strand by the linker.

**12.** The siRNA conjugate according to claim 10, wherein the linker is of the following structure: $-(L^A)_nL^C-L^B-$, wherein n is an integer having the value between 1 and 3;
$L^A$ is a chain moiety comprising amide bond, which is linked to the N-acetylgalactosamine molecule and the $L^C$ moiety through ether bond, and the structure thereof is as follows:

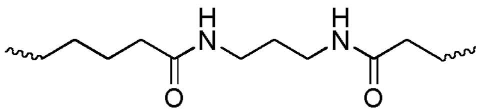
;

$L^B$ is a chain moiety comprising amide bond, which is linked to the $L^C$ moiety through amide bond and linked to the siRNA moiety through phosphoester bond, and the structure thereof is as follows:

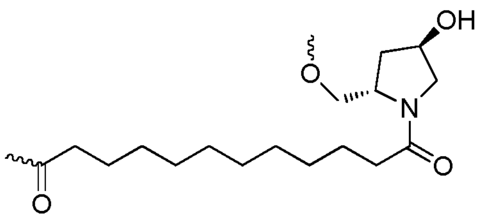
;

and
$L^C$ is a bivalent to tetravalent linking group based on hydroxymethyl aminomethane, dihydroxymethyl aminomethane or trihydroxymethyl aminomethane, one end of which is linked to 1-3 $L^A$ moiety(moieties) through ether bond via oxygen atom; and the other end of which is linked to the $L^B$ moiety through amide bond via nitrogen atom.

**13.** The siRNA conjugate according to claim 10, wherein the structure of the siRNA conjugate is as follows:

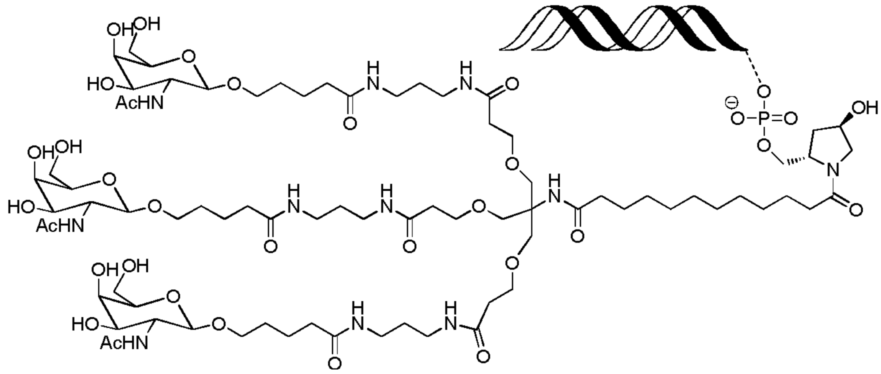

wherein the conjugating molecule is covalently conjugated to the siRNA at the 3'-end of the sense strand, and the molar ratio of the siRNA to N-acetylgalactosamine is 1: 3.

**14.** A kit comprising the siRNA according to claim 1 and/or the pharmaceutical composition according to claim 3 and/or the siRNA conjugate according to claim 9.

**15.** A use of the siRNA according to claim 1 or 2 and/or the pharmaceutical composition according to claim 3 and/or the siRNA conjugate according to claim 9 in the preparation of a drug for treating and/or preventing hepatitis B.

**16.** The siRNA according to claim 1 or 2 and/or the pharmaceutical composition according to any one of claims 3-8 and/or the siRNA conjugate according to any one of claims 9-13 for use in inhibiting the expression of HBV gene in hepatitis cells infected with chronic HBV.

## Patentansprüche

**1.** siRNA mit den nachfolgenden Sequenzen:

Sense-Strang 5'-CmCmUmUmGAGGCmAUmACmUmUmCmAAA-dTdT-3';
Antisense-Strang 5'-UfUmUfGAAGUfAUGCCUfCAAGG-dTdT-3',
wobei die Großbuchstaben C, G, U, A und T die Basenzusammensetzung des Nukleotids darstellen; der Kleinbuchstabe d anzeigt, dass das eine Nukleotid auf der rechten Seite des Buchstabens d ein Desoxyribonukleotid ist; der Kleinbuchstabe m anzeigt, dass die 2'-Hydroxylgruppe der Ribosegruppe des einen Nukleotids auf der linken Seite des Buchstabens m durch eine Methoxygruppe substituiert ist; und der Kleinbuchstabe f anzeigt, dass die 2'-Hydroxylgruppe der Ribosegruppe des einen Nukleotids auf der linken Seite des Buchstabens f durch Fluor substituiert ist.

**2.** siRNA nach Anspruch 1, wobei die Phosphodiesterbindung zwischen dem Überhang dTdT am 3'-Ende des Sense-Strangs und/oder des Antisense-Strangs der siRNA durch eine Phosphorothioatdiesterbindung ersetzt ist.

**3.** Pharmazeutische Zusammensetzung, umfassend die siRNA nach Anspruch 1 und einen pharmazeutisch annehmbaren Träger.

**4.** Pharmazeutische Zusammensetzung nach Anspruch 3, wobei der pharmazeutisch annehmbare Träger eine Lipidmischung ist, bestehend aus einer Amin-enthaltenden Verbindung, einem Cholesterin und einem pegylierten Lipid, wobei die Amin-enthaltende Verbindung eine Verbindung, die durch die Formel I dargestellt wird, oder ein pharmazeutisch annehmbares Salz davon ist:

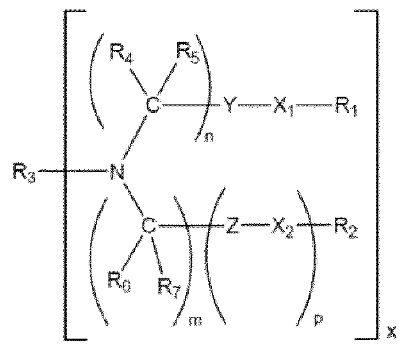

Formel I

wobei:

jedes von $X_1$ und $X_2$ unabhängig ausgewählt ist aus O, S, N-A oder C-A, wobei A Wasserstoff oder eine $C_1$-$C_{20}$-Kohlenwasserstoffkette ist;
jedes von Y und Z unabhängig ausgewählt ist aus C=O, C=S, S=O, CH-OH oder $SO_2$;
jedes von $R_1$, $R_2$, $R_3$, $R_4$, $R_5$, $R_6$ und $R_7$ unabhängig ausgewählt ist aus Wasserstoff; einer zyklischen oder azyklischen, substituierten oder unsubstituierten, verzweigten oder unverzweigten aliphatischen Gruppe; einer zyklischen oder azyklischen, substituierten oder unsubstituierten, verzweigten oder unverzweigten heteroaliphatischen Gruppe; einer substituierten oder unsubstituierten, verzweigten oder unverzweigten Acyl-Gruppe; einer substituierten oder unsubstituierten, verzweigten oder unverzweigten Aryl-Gruppe, einer substituierten oder unsubstituierten, verzweigten oder unverzweigten Heteroaryl-Gruppe;
x eine ganze Zahl mit einem Wert zwischen 1 und 10 ist;
n eine ganze Zahl mit einem Wert zwischen 1 und 3 ist, m eine ganze Zahl mit einem Wert zwischen 0 und 20 ist, p eine ganze Zahl mit einem Wert von 0 oder 1 ist, wobei, wenn m=p=O ist, dann ist $R_2$ Wasserstoff,
mit dem weiteren Vorbehalt, dass, wenn mindestens eines von n oder m den Wert 2 hat, $R_3$ und Stickstoff in der Formel I eine Struktur bilden, wie sie durch die Formel II oder die Formel III dargestellt wird:

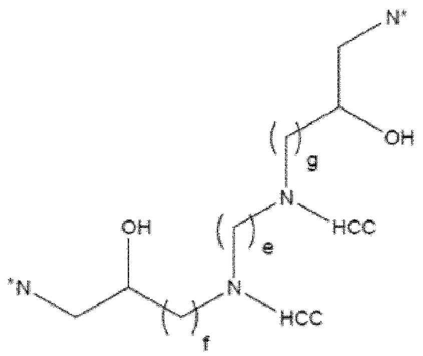

Formel II,

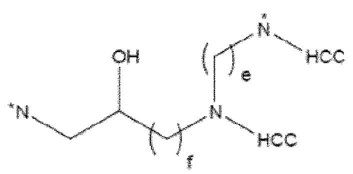

Formel III,

wobei jedes von g, e und f unabhängig eine ganze Zahl mit einem Wert zwischen 1 und 6 ist, "HCC" eine Kohlenwasserstoffkette symbolisiert und jedes * das Stickstoffatom in der Formel I anzeigt.

**5.** Pharmazeutische Zusammensetzung nach Anspruch 4, wobei
die Amin-enthaltende Verbindung die Amin-enthaltende Verbindung 72 oder die Amin-enthaltende Verbindung 87 wie folgt ist:

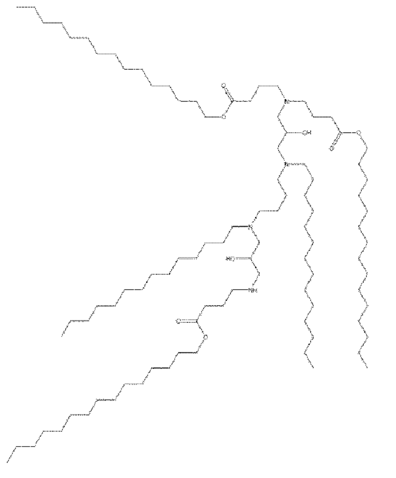

72 ;

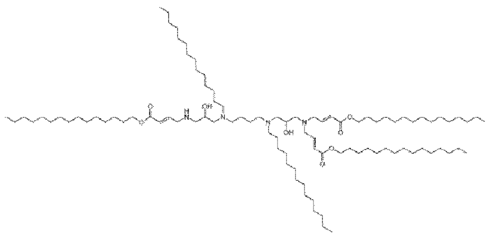

87 ,

und das pegylierte Lipid 1 ,2-Dipalmitoyl-sn-glycero-3-phosphoethanolamin-N-[methoxy(polyethylenglycol)-2000] ist.

**6.** Pharmazeutische Zusammensetzung nach Anspruch 4, wobei die Molprozentsätze der Amin-enthaltenden Verbindung, des Cholesterins und des pegylierten Lipids in dem pharmazeutisch annehmbaren Träger 19,7 bis 80 % für die Amin-enthaltende Verbindung, 19,7 bis 80 % für das Cholesterin und 0,3 % - 50 % für das pegylierte Lipid betragen, vorzugsweise sind die Molprozentsätze der Amin-enthaltenden Verbindung, des Cholesterins und des pegylierten Lipids in dem pharmazeutisch annehmbaren Träger 50 %-70 % für die Amin-enthaltende Verbindung, 20 %-40 % für das Cholesterin und 3 %-20 % für das pegylierte Lipid.

**7.** Pharmazeutische Zusammensetzung nach Anspruch 5, wobei die Molprozentsätze der Amin-enthaltenden Verbindung 87, des Cholesterins und 1 ,2-Dipalmitoyl-sn-glycero-3-phosphoethanolamin-N-[methoxy(polyethylenglycol)-2000] in dem pharmazeutisch annehmbaren Träger 19,7 %-80 % für die Amin-enthaltende Verbindung 87, 19,7 %-80 % für das Cholesterin und 0,3 %-50 % für 1 ,2-Dipalmitoyl-sn-glycero-3-phosphoethanolamin-N-[methoxy(polyethylenglycol)-2000] betragen,
vorzugsweise betragen die Molprozentsätze der Amin-enthaltenden Verbindung 87, des Cholesterins und 1 ,2-Dipalmitoyl-sn-glycero-3-phosphoethanolamin-N-[methoxy(polyethylenglycol)-2000] im pharmazeutisch annehmbaren Träger 50 bis 70 % für die Amin-enthaltende Verbindung 87, 20 %-40 % für das Cholesterin und 3 %-20 % für 1 ,2-Dipalmitoyl-sn-glycero-3-phosphoethanolamin-N-[methoxy(polyethylenglycol)-2000].

**8.** Pharmazeutische Zusammensetzung nach Anspruch 3, wobei das Gewichtsverhältnis der siRNA zum pharmazeutisch annehmbaren Träger 1: (1-50) beträgt.

**9.** siRNA-Konjugat, erhalten durch Konjugieren der siRNA nach Anspruch 1 an ein pharmazeutisch annehmbares Konjugationsmolekül, wobei das Konjugationsmolekül ein pharmazeutisch annehmbares Targeting-Molekül und einen optionalen Linker umfasst.

**10.** siRNA-Konjugat nach Anspruch 9, wobei das pharmazeutisch annehmbare Targeting-Molekül ein Ligand für den Asialoglykoprotein-Rezeptor ist, vorzugsweise ist das pharmazeutisch annehmbare Targeting-Molekül Galactose oder N-Acetylgalactosamin.

**11.** siRNA-Konjugat nach Anspruch 10, wobei die Galactose oder das N-Acetylgalactosamin durch den Linker kovalent an die siRNA konjugiert ist, vorzugsweise ist die Galactose oder das N-Acetylgalactosamin am 3'-Ende des Sense-Strangs durch den Linker kovalent an die siRNA konjugiert.

**12.** siRNA-Konjugat nach Anspruch 10, wobei der Linker die folgende Struktur aufweist: $-(L^A)_nL^C-L^B-$, wobei n eine ganze Zahl mit einem Wert zwischen 1 und 3 ist;
$L^A$ eine Ketteneinheit ist, umfassend eine Amidbindung, die durch eine Etherbindung mit dem N-Acetylgalactosamin-Molekül und der $L^C$-Einheit verbunden ist, und die Struktur davon wie folgt ist:

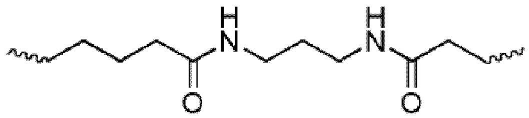
;

$L^B$ eine Ketteneinheit ist, umfassend eine Amidbindung, die durch die Amidbindung mit der $L^C$-Einheit verbunden ist und über eine Phosphoesterbindung mit der siRNA-Einheit verbunden ist, und die Struktur davon wie folgt ist:

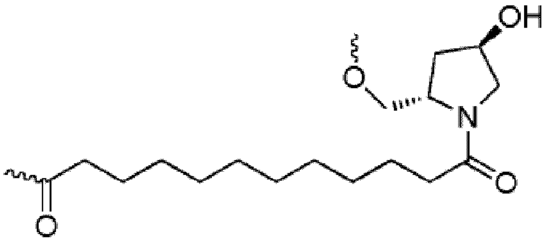
;

und
$L^C$ eine bivalente bis tetravalente Verbindungsgruppe ist, beruhend auf Hydroxymethylaminomethan, Dihydroxymethylaminomethan oder Trihydroxymethylaminomethan, wobei ein Ende davon durch eine Etherbindung mittels eines Sauerstoffatoms mit 1-3 $L^A$-Einheit(en) verbunden ist; und wobei das andere Ende davon durch eine Amidbindung mittels eines Stickstoffatoms mit der $L^B$-Einheit verbunden ist.

**13.** siRNA-Konjugat nach Anspruch 10, wobei die Struktur des siRNA-Konjugats wie folgt ist:

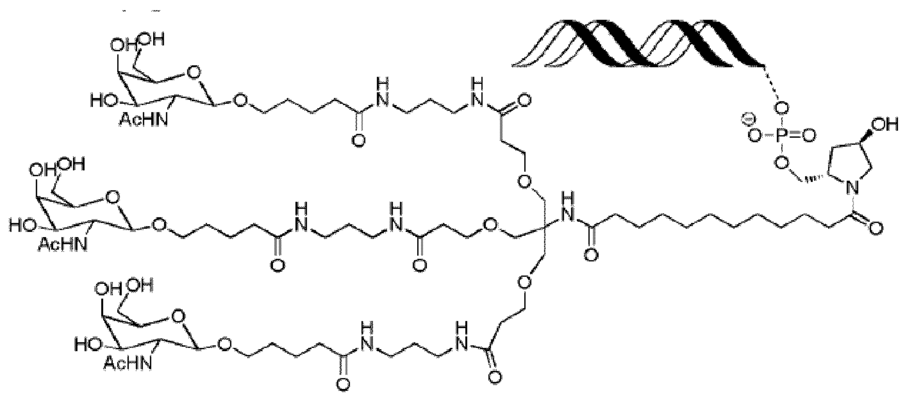

wobei das Konjugationsmolekül am 3'-Ende des Sense-Strangs kovalent an die siRNA konjugiert ist, und das molare Verhältnis der siRNA zu N-Acetylgalactosamin 1: 3 beträgt.

**14.** Kit, umfassend die siRNA nach Anspruch 1 und/oder die pharmazeutische Zusammensetzung nach Anspruch 3 und/oder das siRNA-Konjugat nach Anspruch 9.

**15.** Verwendung der siRNA nach Anspruch 1 oder 2 und/oder der pharmazeutischen Zusammensetzung nach Anspruch 3 und/oder des siRNA-Konjugats nach Anspruch 9 bei der Herstellung eines Medikaments zur Behandlung und/oder Prävention von Hepatitis B.

16. siRNA nach Anspruch 1 oder 2 und/oder die pharmazeutische Zusammensetzung nach einem der Ansprüche 3-8 und/oder das siRNA-Konjugat nach einem der Ansprüche 9-13 zur Verwendung bei der Hemmung der Expression des HBV-Gens in Hepatitis-Zellen, die mit chronischem HBV infiziert sind.

**Revendications**

1. siARN, avec les séquences suivantes :

brin sens 5'-CmCmUmUmGAGGCmAUmACmUmUmCmAAA-dTdT-3' ;
brin antisens 5'-UfUmUfGAAGUfAUGCCUfCAAGG-dTdT-3',
dans lequel les lettres majuscules C, G, U, A et T représentent la composition de base du nucléotide ; la lettre minuscule d indique que l'un nucléotide sur la droite de la lettre d est un désoxyribonucléotide ; la lettre minuscule m indique que le groupe 2'-hydroxyle du groupe ribose de l'un nucléotide sur la gauche de la lettre m est substitué avec un groupe méthoxy ; et la lettre minuscule f indique que le groupe 2'-hydroxyle du groupe ribose de l'un nucléotide sur la gauche de la lettre f est substitué avec du fluor.

2. siARN selon la revendication 1, dans lequel la liaison phosphodiester entre le dTdT débordant à l'extrémité 3' du brin sens et/ou du brin antisens du siARN est remplacé par une liaison diester de phosphorothioate.

3. Composition pharmaceutique, comprenant le siARN selon la revendication 1 et un vecteur pharmaceutiquement acceptable.

4. Composition pharmaceutique selon la revendication 3, dans laquelle le vecteur pharmaceutiquement acceptable est un mélange de lipide constitué d'un composé contenant de l'amine, d'un cholestérol, et d'un lipide pégylé, dans laquelle le composé contenant de l'amine est un composé représenté par la formule I ou un sel pharmaceutiquement acceptable de celui-ci :

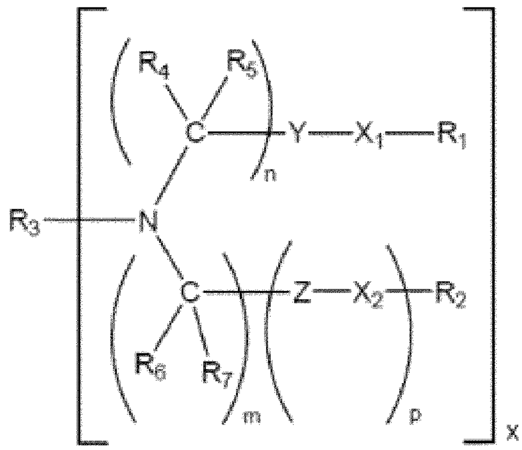
formule I

dans laquelle

chacun de $X_1$ et de $X_2$ est indépendamment sélectionné parmi O, S, N-A ou C-A, dans laquelle A est hydrogène ou une chaîne hydrocarbonée $C_1$-$C_{20}$ ;
chacun de Y et de Z est indépendamment sélectionné parmi C=O, C=S, S=O, CH-OH ou $SO_2$;
chacun de $R_1$, de $R_2$, de $R_3$, de $R_4$, de $R_5$, de $R_5$ et de $R_7$ est indépendamment sélectionné parmi : hydrogène ; un groupe aliphatique ramifié ou non ramifié, substitué ou non substitué, cyclique ou acyclique ; un groupe hétéroaliphatique ramifié ou non ramifié, substitué ou non substitué, cyclique ou acyclique ; un groupe acyle ramifié ou non ramifié, substitué ou non substitué ; un groupe aryle ramifié ou non ramifié, substitué ou non substitué ; un groupe hétéroaryle ramifié ou non ramifié, substitué ou non substitué ;
x est un nombre entier relatif ayant la valeur entre 1 et 10 ;
n est un nombre entier relatif ayant la valeur entre 1 et 3, m est un nombre entier relatif ayant la valeur entre 0 et 20, p est un nombre entier relatif ayant la valeur de 0 ou 1, dans laquelle si m=p=0, alors $R_2$ est hydrogène, avec la condition supplémentaire que, si au moins un de n ou de m a la valeur de 2, alors $R_3$ et azote dans la

formule I forment une structure telle que représentée par la formule II ou la formule III :

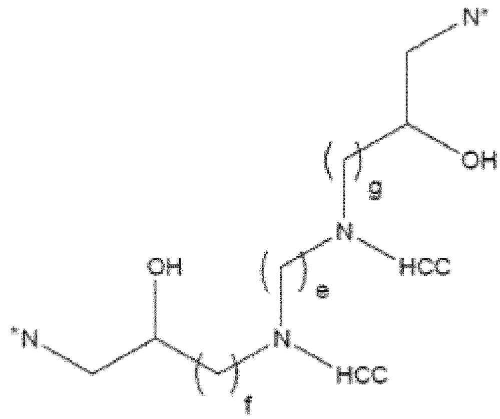

formule II ;

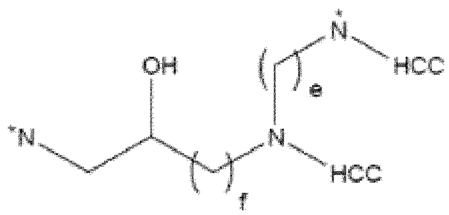

formule III

dans laquelle chacun de g, d'e et de f est indépendamment un nombre entier relatif ayant la valeur entre 1 et 6, « HCC » symbolise une chaîne hydrocarbonée, et chaque * indique l'atome d'azote dans la formule I.

**5.** Composition pharmaceutique selon la revendication 4, dans laquelle le composé contenant de l'amine est le composé, contenant de l'amine, 72 ou le composé, contenant de l'amine, 87 comme suit :

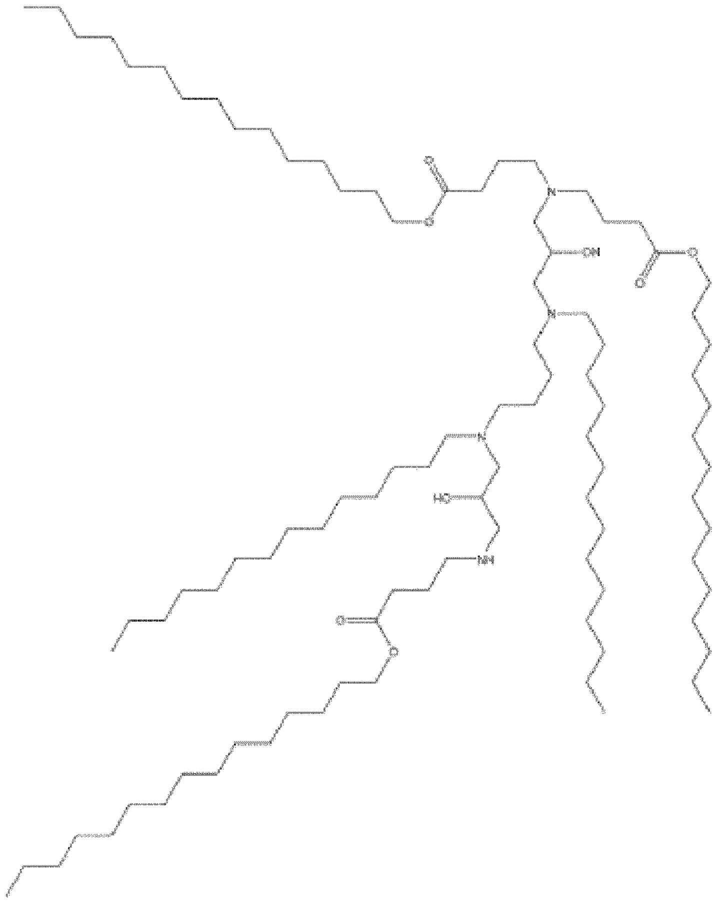

**72** ;

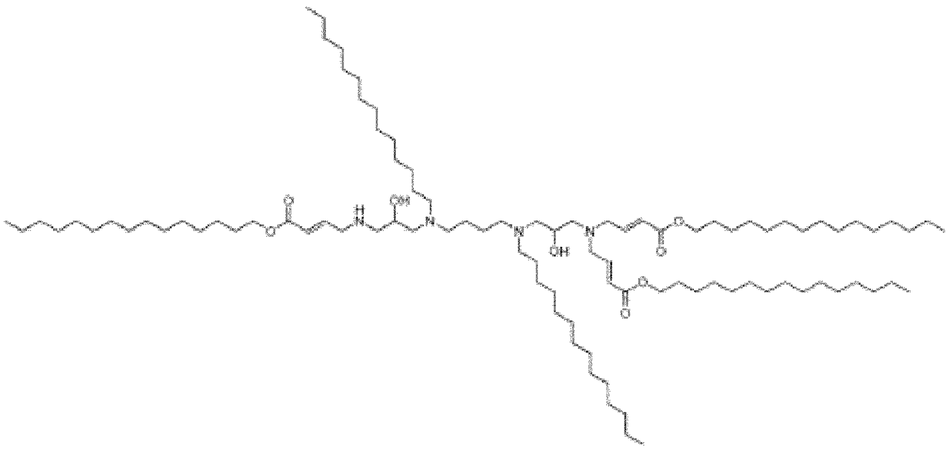

**87** ,

et le lipide pégylé est 1,2-dipalmitoyl-sn-glycéro-3-phosphoéthanolamine-N-[méthoxy(polyéthylèneglycol)-2000].

6. Composition pharmaceutique selon la revendication 4, dans laquelle les pourcentages molaires du composé contenant de l'amine, du cholestérol, et du lipide pégylé dans le vecteur pharmaceutiquement acceptable sont de 19,7 % à 80 % pour le composé contenant de l'amine, de 19,7 % à 80 % pour le cholestérol, et de 0,3 % à 50 % pour le lipide pégylé,
de préférence, les pourcentages molaires du composé contenant de l'amine, du cholestérol, et du lipide pégylé dans le vecteur pharmaceutiquement acceptable sont de 50 % à 70 % pour le composé contenant de l'amine, de

20 % à 40 % pour le cholestérol, et de 3 % à 20 % pour le lipide pégylé.

**7.** Composition pharmaceutique selon la revendication 5, dans laquelle les pourcentages molaires du composé, contenant de l'amine, 87, du cholestérol, et du 1,2-dipalmitoyl-*sn*-glycéro-3-phosphoéthanolamine-N-[méthoxy(polyéthylèneglycol)-2000] dans le vecteur pharmaceutiquement acceptable sont de 19,7 % à 80 % pour le composé, contenant de l'amine, 87, de 19,7 % à 80 % pour le cholestérol, et de 0,3 % à 50 % pour le 1,2-dipalmitoyl-*sn*-glycéro-3-phosphoéthanolamine-N-[méthoxy(polyéthylèneglycol)-2000],
de préférence, les pourcentages molaires du composé, contenant de l'amine, 87, du cholestérol, et du 1,2-dipalmitoyl-*sn*-glycéro-3-phosphoéthanolamine-N-[méthoxy(polyéthylèneglycol)-2000] dans le vecteur pharmaceutiquement acceptable sont de 50 % à 70 % pour le composé, contenant de l'amine, 87, de 20 % à 40 % pour le cholestérol, et de 3 % à 20 % pour le 1,2-dipalmitoyl-*sn*-glycéro-3-phosphoéthanolamine-N-[méthoxy(polyéthylèneglycol)-2000].

**8.** Composition pharmaceutique selon la revendication 3, dans laquelle le rapport pondéral du siARN par rapport au vecteur pharmaceutiquement acceptable est 1 : (1-50).

**9.** Conjugué de siARN obtenu en conjuguant le siARN selon la revendication 1 à une molécule de conjugaison pharmaceutiquement acceptable, dans lequel la molécule de conjugaison comprend une molécule de ciblage pharmaceutiquement acceptable et un lieur optionnel.

**10.** Conjugué de siARN selon la revendication 9, dans lequel la molécule de ciblage pharmaceutiquement acceptable est un ligand pour récepteur asialoglycoprotéique, de préférence, la molécule de ciblage pharmaceutiquement acceptable est galactose ou N-acétylgalactosamine.

**11.** Conjugué de siARN selon la revendication 10, dans lequel le galactose ou la N-acétylgalactosamine est conjugué de façon covalente au siARN par le lieur, de préférence, le galactose ou la N-acétylgalactosamine est conjugué de façon covalente au siARN à l'extrémité 3' du brin sens par le lieur.

**12.** Conjugué de siARN selon la revendication 10, dans lequel le lieur est de la structure suivante : $-(L^A)_nL^C-L^B-$, dans lequel
n est un nombre entier relatif ayant la valeur entre 1 et 3 ;
$L^A$ est un groupe caractéristique en chaîne comprenant une liaison amide, qui est lié à la molécule N-acétylgalactosamine et au groupe caractéristique $L^C$ par l'intermédiaire de liaison éther, et la structure de celui-ci est comme suit :

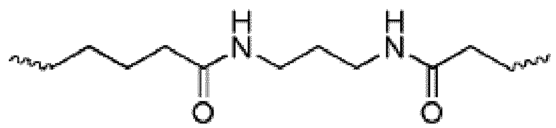
;

$L^B$ est un groupe caractéristique en chaîne comprenant liaison amide, qui est lié au groupe caractéristique $L^C$ par l'intermédiaire de liaison amide et lié au groupe caractéristique siARN par l'intermédiaire de liaison phosphoester, et la structure de celui-ci est comme suit :

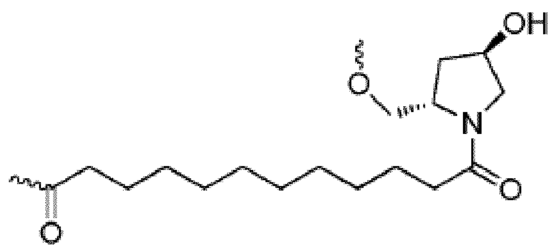
;

et
$L^C$ est un groupe liant bivalent à tétravalent sur la base d'aminométhane d'hydroxyméthyle, d'aminométhane de dihydroxyméthyle ou d'aminométhane de trihydroxyméthyle, dont une extrémité est liée à 1 à 3 groupe(s) caractéristique(s) $L^A$ par l'intermédiaire de liaison éther grâce à un atome d'oxygène ; et dont l'autre extrémité est liée au groupe caractéristique $L^B$ par l'intermédiaire de liaison amide grâce à un atome d'azote.

13. Conjugué de siARN selon la revendication 10, dans lequel la structure du conjugué de siARN est comme suit :

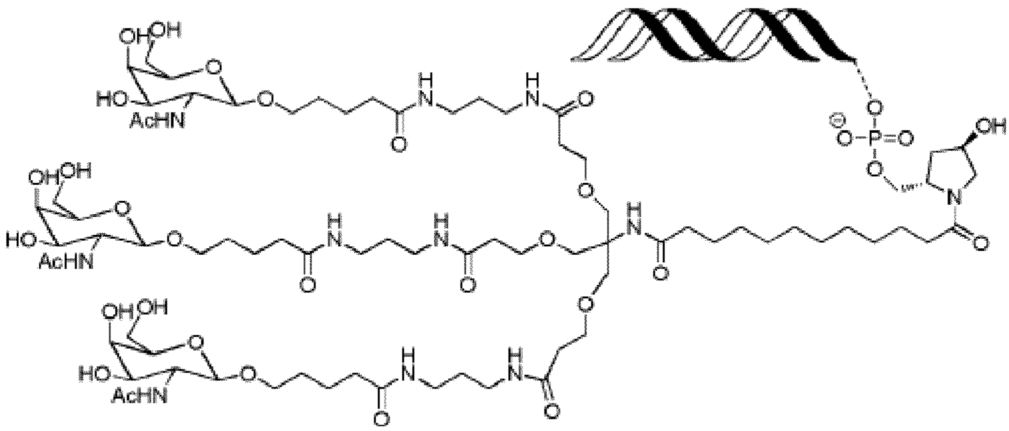

,

dans lequel la molécule de conjugaison est conjuguée de façon covalente au siARN à l'extrémité 3' du brin sens, et le rapport molaire de siARN par rapport à N-acétylgalactosamine est 1 : 3.

14. Kit comprenant le siARN selon la revendication 1 et/ou la composition pharmaceutique selon la revendication 3 et/ou le conjugué de siARN selon la revendication 9.

15. Utilisation du siARN selon la revendication 1 ou 2 et/ou de la composition pharmaceutique selon la revendication 3 et/ou du conjugué de siARN selon la revendication 9 dans la préparation d'un médicament pour le traitement et/ou la prévention d'hépatite B.

16. siARN selon la revendication 1 ou 2 et/ou composition pharmaceutique selon l'une quelconque des revendications 3 à 8 et/ou conjugué de siARN selon l'une quelconque des revendications 9 à 13 pour l'utilisation dans l'inhibition de l'expression de gène HBV dans des cellules d'hépatite infectées avec HBV chronique.

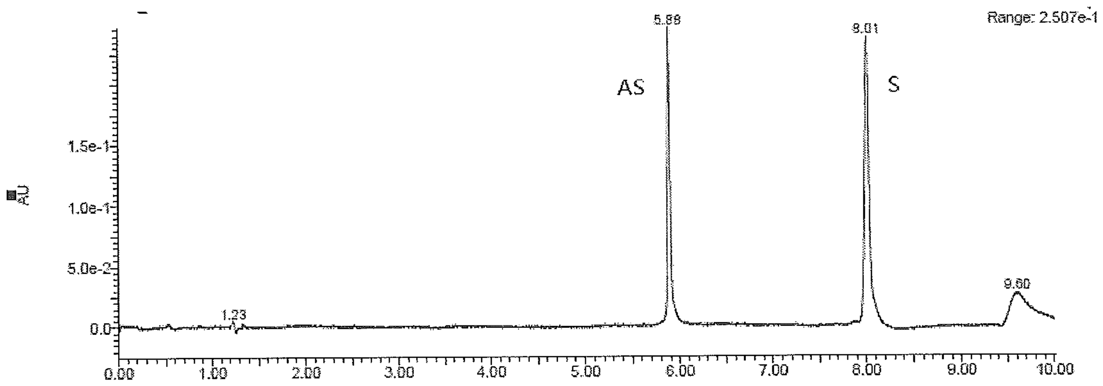


FIG. 1a

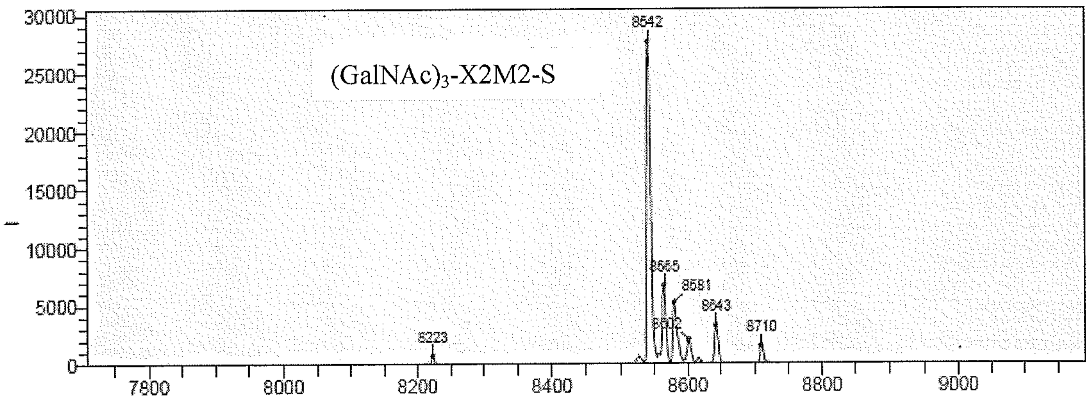


FIG. 1b

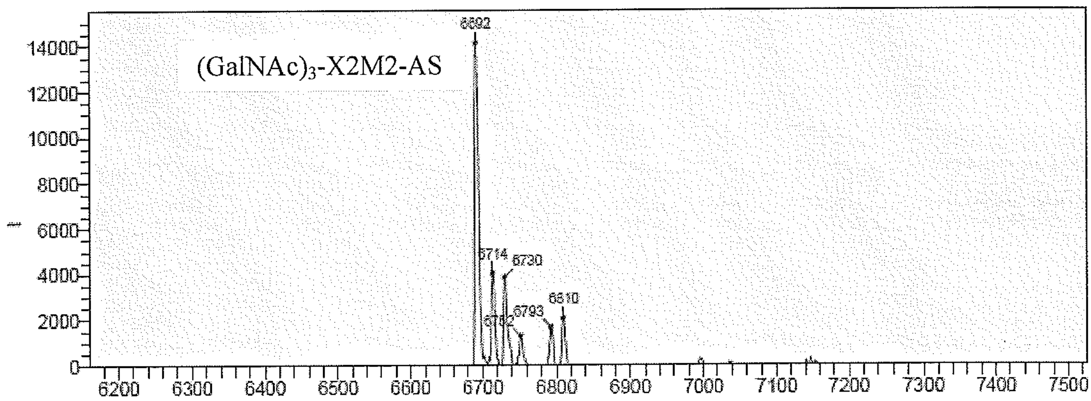


FIG. 1c

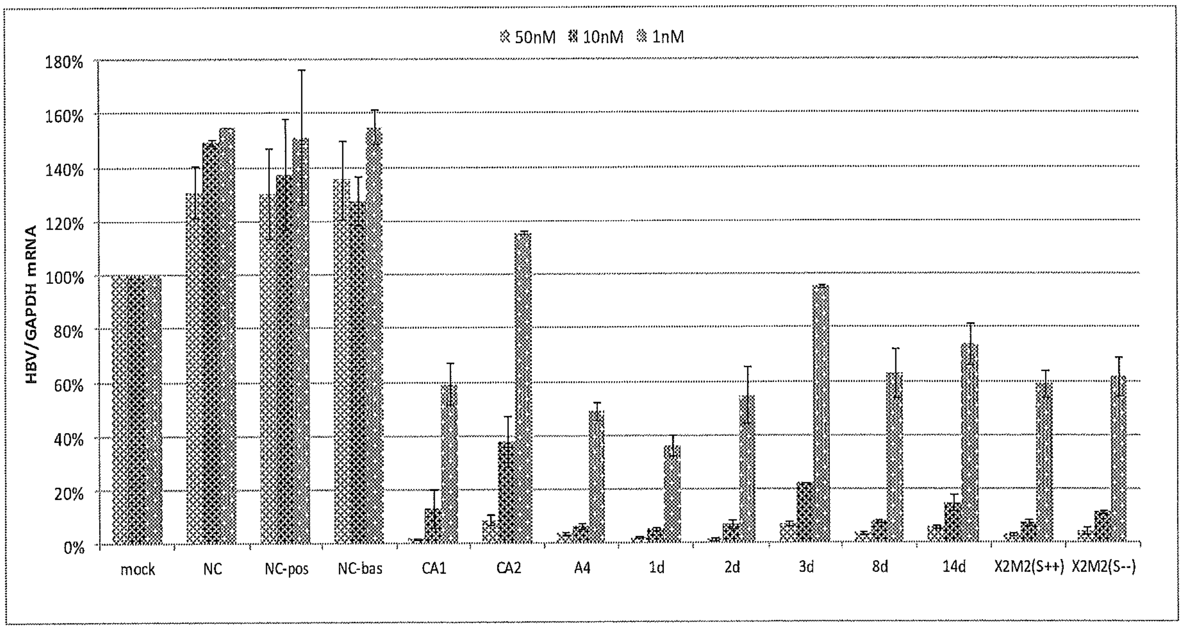
50nM 10nM 1nM
180%
160%
140%
120%
100%
80%
60%
40%
20%
0%
HBV/GAPDH mRNA
mock
NC
NC-pos
NC-bas
CA1
CA2
A4
1d
2d
3d
8d
14d
X2M2(S++)
X2M2(S--)

FIG. 2

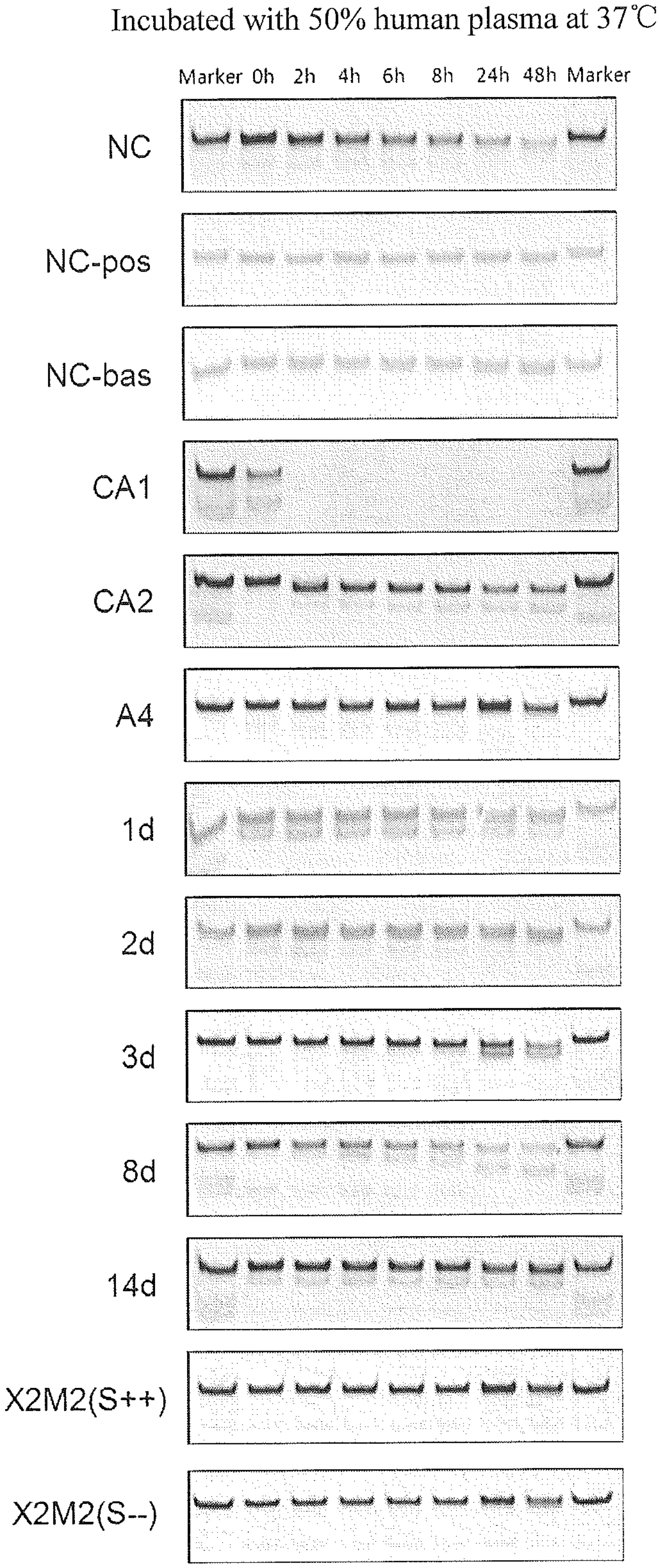
Incubated with 50% human plasma at 37℃
Marker 0h 2h 4h 6h 8h 24h 48h Marker
NC
NC-pos
NC-bas
CA1
CA2
A4
1d
2d
3d
8d
14d
X2M2(S++)
X2M2(S--)

FIG. 3

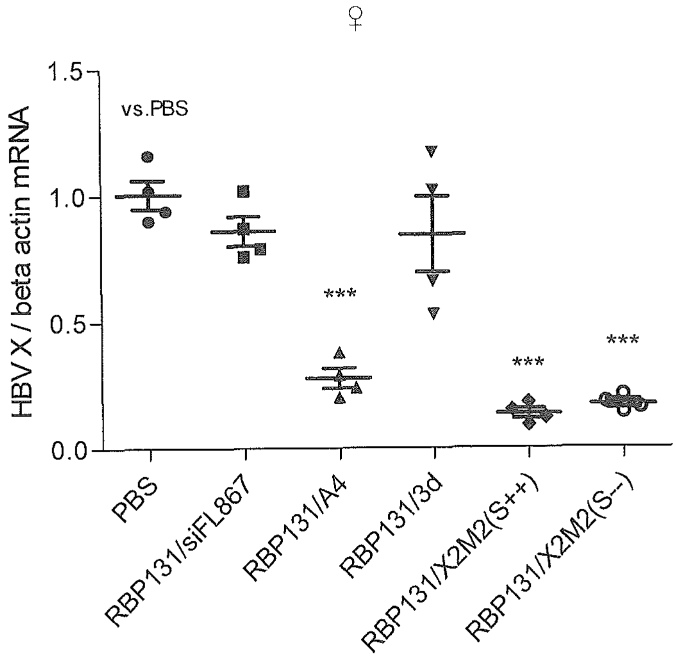
♀
HBV X / beta actin mRNA
1.5
1.0
0.5
0.0
vs.PBS
***
***
***
PBS
RBP131/siFL867
RBP131/A4
RBP131/3d
RBP131/X2M2(S++)
RBP131/X2M2(S--)

FIG. 4

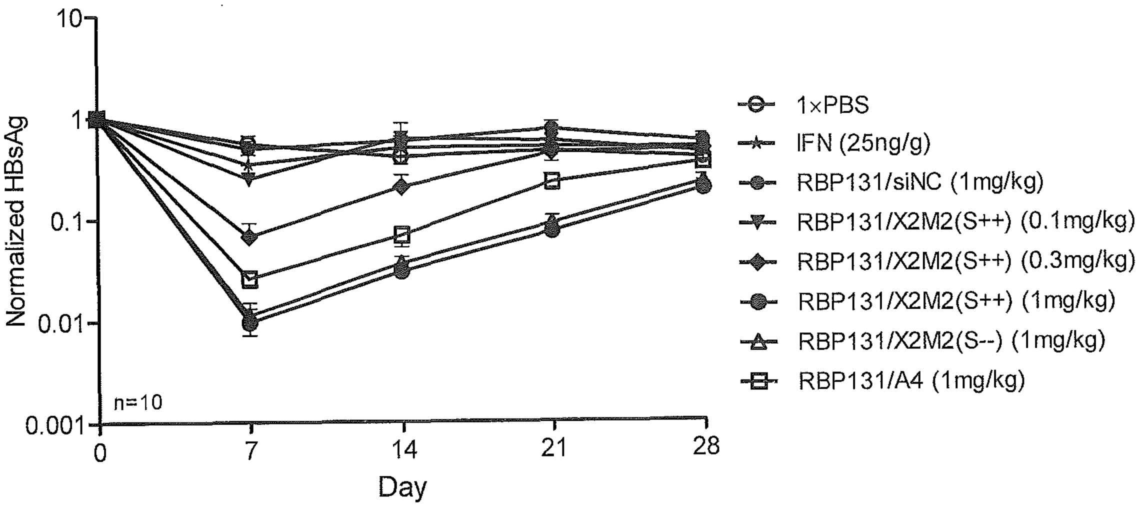
Normalized HBsAg
10
1
0.1
0.01
0.001
n=10
0
7
14
21
28
Day
1×PBS
IFN (25ng/g)
RBP131/siNC (1mg/kg)
RBP131/X2M2(S++) (0.1mg/kg)
RBP131/X2M2(S++) (0.3mg/kg)
RBP131/X2M2(S++) (1mg/kg)
RBP131/X2M2(S--) (1mg/kg)
RBP131/A4 (1mg/kg)

FIG. 5

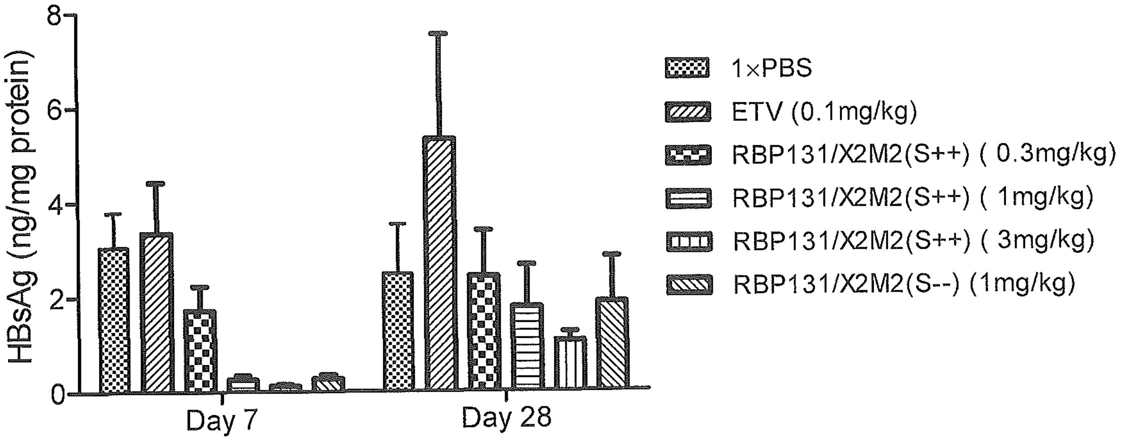
HBsAg (ng/mg protein)
8
6
4
2
0
Day 7
Day 28
1×PBS
ETV (0.1mg/kg)
RBP131/X2M2(S++) ( 0.3mg/kg)
RBP131/X2M2(S++) ( 1mg/kg)
RBP131/X2M2(S++) ( 3mg/kg)
RBP131/X2M2(S--) (1mg/kg)

FIG. 6

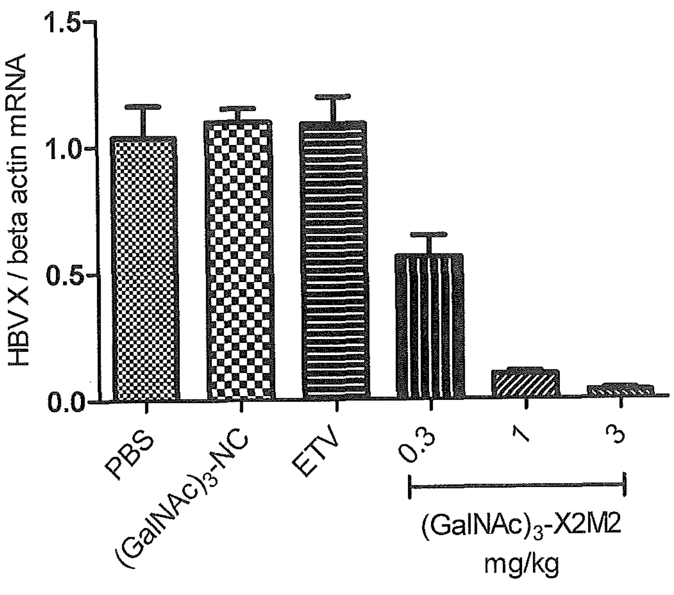
HBV X / beta actin mRNA
1.5
1.0
0.5
0.0
PBS
(GalNAc)3-NC
ETV
0.3
1
3
(GalNAc)3-X2M2
mg/kg

FIG. 7

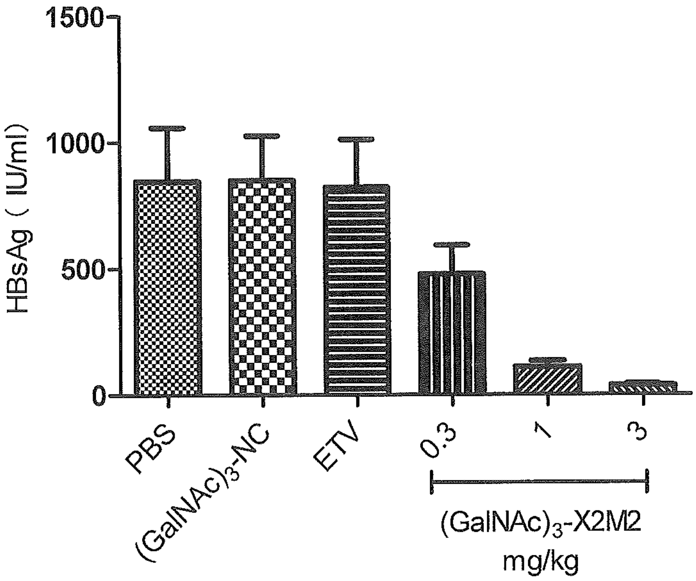
1500
1000
500
0
HBsAg ( IU/ml)
PBS
(GalNAc)3-NC
ETV
0.3
1
3
(GalNAc)3-X2M2
mg/kg

FIG. 8

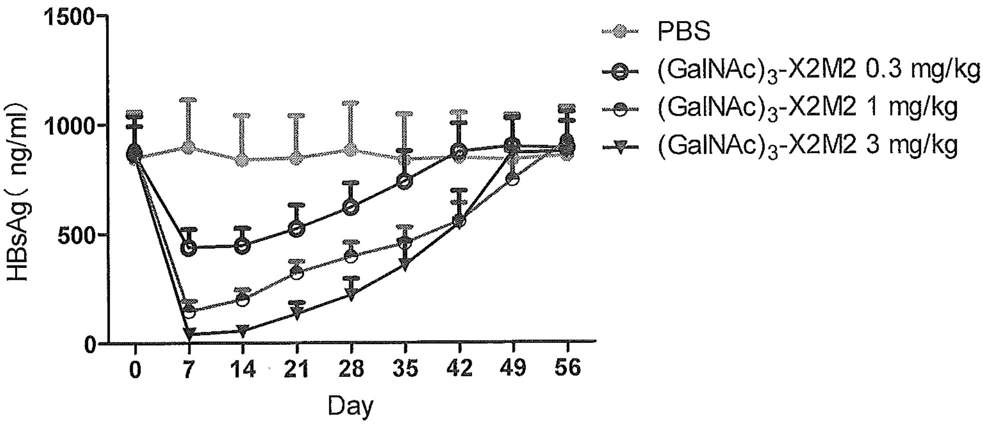
1500
1000
500
0
HBsAg( ng/ml)
0
7
14
21
28
35
42
49
56
Day
PBS
(GalNAc)3-X2M2 0.3 mg/kg
(GalNAc)3-X2M2 1 mg/kg
(GalNAc)3-X2M2 3 mg/kg

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102140461 B
- CN 201180060664
- WO 2014025805 A1


### Non-patent literature cited in the description


- **WATTS, J.K. ; G.F. DELEAVEY ; M.J. DAMHA.** Chemically modified siRNA: tools and applications. *Drug Discov Today,* 2008, vol. 13 (19-20), 842-55
- Molecular Cloning. Cold Spring Harbor Laboratory Press, 1989
- **REN J. et al.** *J. Medical Virology,* 2006, vol. 78, 551-560
- **PENG XH. et al.** *World J. Gastroenterol.,* 2015, vol. 21 (12), 3527-3536
- *Biomaterials,* June 2012, vol. 33 (18), 4653-64
- *Biomaterials,* April 2013, vol. 34 (12), 3120-31
- *Biomaterials,* June 2012, vol. 33 (18), 46 53-64
- *Biomaterials,* April 2013, vol. 34 (12), 3 120-31

SEQUENCE LISTING

<110> SUZHOU RIBO LIFE SCIENCE CO., LTD

<120> SIRNA, PHARMACEUTICAL COMPOSITION AND CONJUGATE WHICH CONTAIN SIRNA, AND USES THEREOF

<130>

<160> 17

<170> PatentIn version 3.3

<210> 1
<211> 21
<212> RNA/DNA
<213> Artificial Sequence: siRNA sense strand

<400> 1
ccuugaggca uacuucaaat t 21

<210> 2
<211> 21
<212> RNA/DNA
<213> Artificial Sequence: siRNA antisense strand

<400> 2
uuugaaguau gccucaaggt t 21

<210> 3
<211> 19
<212> RNA
<213> HBV mRNA

<400> 3
ccuugaggca uacuucaaa 19

<210> 4
<211> 21
<212> RNA/DNA
<213> Artificial Sequence: siRNA sense strand

<400> 4
uucuccgaac gugucacgut t 21

<210> 5
<211> 21

<212> RNA/DNA
<213> Artificial Sequence: siRNA antisense strand

<400> 5
acgugacacg uucggagaat t 21

<210> 6
<211> 20
<212> DNA
<213> Artificial Sequence: synthesized primer

<400> 6
ccgtctgtgc cttctcatct 20

<210> 7
<211> 20
<212> DNA
<213> Artificial Sequence: synthesized primer

<400> 7
taatctcctc ccccaactcc 20

<210> 8
<211> 20
<212> DNA
<213> Artificial Sequence: synthesized primer

<400> 8
agaaggctgg ggctcatttg 20

<210> 9
<211> 20
<212> DNA
<213> Artificial Sequence: synthesized primer

<400> 9
aggggccatc cacagtcttc 20

<210> 10
<211> 21
<212> RNA/DNA
<213> Artificial Sequence: siRNA sense strand

<400> 10
cccuauucuc cuucuucgct t 21

<210> 11
<211> 21
<212> RNA/DNA
<213> Artificial Sequence: siRNA antisense strand

<400> 11
gcgaagaagg agaauagggt t 21

<210> 12
<211> 24
<212> DNA
<213> Artificial Sequence: synthesized primer

<400> 12
agcttctttg cagctccttc gttg 24

<210> 13
<211> 24
<212> DNA
<213> Artificial Sequence: synthesized primer

<400> 13
ttctgaccca ttcccaccat caca 24

<210> 14
<211> 24
<212> DNA
<213> Artificial Sequence: synthesized primer

<400> 14
ttccagccat gggcaacttt acct 24

<210> 15
<211> 24
<212> DNA
<213> Artificial Sequence: synthesized primer

<400> 15
tactgcaggg cgtcagtgac aaat 24

<210> 16
<211> 23
<212> RNA/DNA
<213> Artificial Sequence: siRNA sense strand

<400> 16
gucaucacac ugaauaccaa utt 23

<210> 17
<211> 25
<212> RNA/DNA
<213> Artificial Sequence: siRNA antisense strand

<400> 17
auugguauuc agugugauga cactt 25